# EUROPEAN PATENT APPLICATION

(11) **EP 4 549 435 A1**
(43) Date of publication of application: **07.05.2025**
(21) Application number: 23830516.3
(22) Date of filing: 30.06.2023
(51) Int. Cl.: C07D 401/06, C07D 401/02, A61K 31/454, A61P 37/00, A61P 13/12

(54) **PHARMACEUTICALLY ACCEPTABLE SALT AND CRYSTAL FORM OF NITROGEN-CONTAINING BRIDGE HETEROCYCLIC DERIVATIVE, AND METHOD FOR PREPARING SAME**

(30) Priority: 30.06.2022 CN 202210770229
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: WANG, Lin, Shanghai 200245 (CN); SHAO, Qiyun, Shanghai 200245 (CN); FENG, Jun, Shanghai 200245 (CN); HE, Feng, Shanghai 200245 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent von Kreisler Selting Werner - Partnerschaft von Patent- und Rechtsanwälten mbB
(86) International application number: PCT/CN2023/104908
(87) International publication number: WO 2024/002353

(57) **Abstract**

Provided are a pharmaceutically acceptable salt and a crystal form of a nitrogen-containing bridge heterocyclic derivative, and a method for preparing same. Specifically provided are different salt forms and crystal forms of salts of 4-((1S,3S,5R)-3-ethoxy-8-((5-methoxy-7-methyl-1H-indol-4-yl)methyl)-8-azabicyclo[3.2.1]octyl-1-yl)benzoic acid, and a method for preparing same. The provided crystal forms of salts of 4-((1S,3S,5R)-3-ethoxy-8-((5-methoxy-7-methyl-1H-indol-4-yl)methyl)-8-azabicyclo[3.2.1]octyl-1-yl)benzoic acid have good stability and can be better used for clinical treatment.

## Description

The present application claims priority to Chinese Patent Application No. 2022107702298 filed on June 30, 2022, which is incorporated herein by reference in its entirety.

### TECHNICAL FIELD

The present disclosure pertains to the field of pharmaceutics and relates to a pharmaceutically acceptable salt and a crystal form of a nitrogen-containing bridged heterocyclic derivative and particularly to a pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid, a crystal form thereof, and a preparation method therefor.

### BACKGROUND

Complement is a serum protein that occurs in the serum and tissue fluid of humans and vertebrates. It is thermolabile, has enzyme activity after activation, can mediate immune and inflammatory responses, and can be activated by antigen-antibody complexes or microorganisms, causing lysis or phagocytosis of pathogenic microorganisms.

The complement system is an important regulator of inflammatory responses and tissue damage and consists of more than 20 serum proteins and cell surface proteins. The complement system includes complement innate components and a variety of regulatory proteins. The complement innate components include C1-C9, and the C3 content is the highest. The complement regulatory proteins are further divided into two categories: soluble ones and membrane-bound ones. The soluble complement regulatory proteins include clusterin, S protein, complement factor H-related proteins, and the like. The membrane-bound complement regulatory proteins include membrane cofactor protein (MCP), decay accelerating factor (DAF), complement receptor 1, and the like. In addition, the complement system also includes some complement fragments and complement receptors, such as the C3a receptor and the C5a receptor.

The complement system is activated via three independent and intersecting pathways, namely the classical pathway (CP), the alternative pathway (AP), and the lectin pathway (LP, also known as the MBL (mannan-binding lectin) pathway). In the process of complement activation, a strong biological effect is produced through a series of positive feedback and is involved in the development and progression of disease. C3 convertase is an important component of the first three pathways. It causes production of a range of complement protein fragments and the membrane attack complex (MAC) via a complement activation cascade reaction. C3 convertase cleaves C3 to produce C5 convertase. Subsequently, C5 convertase cleaves C5 to produce C5a and C5b, and C5b binds to C6, C7, C8, and C9 to form C5b-9, i.e., MAC. Abnormalities in the complement pathways will cause lysis of the body's innate normal cells, thereby leading to the development of disease.

Complement factor B (Factor B) is a thermolabile β globin, which can be inactivated by being simply heated at 50 °C for 30 min. It can be cleaved by complement factor D into two fragments: Ba and Bb, and Bb binds to C3b to form C3 convertase of the alternative pathway. Complement factor B, also known as C3 proactivator, is an important component of the complement alternative activation pathway. Complement factor B has a molecular weight of 93 kDa, is present in human blood at a concentration of about 3 µM, and is synthesized primarily in the liver. It is found that complement factor B is also synthesized in the retinal pigment epithelial cells of the eyes.

Glomerulopathy includes IgA nephropathy (IgAN for short), C3G glomerulopathy (C3G for short), membranous glomerulonephritis (MGN for short), and the like. IgAN and MGN are the most common of them. However, there has been some increase in the incidence of rare kidney diseases such as C3 glomerulopathy over the last decade. Glomerulopathy has been found to be closely related to the complement pathways, especially the complement alternative pathway. Currently, there is a lack of clinically effective treatment regimens for primary glomerulonephritis. Medications such as hormones and immunosuppressants (e.g., cyclophosphamide, mycophenolate mofetil, tacrolimus, cyclosporine A, and the traditional Chinese medicine tripterygium glycosides) are typically used. Other medications include blood-pressure-controlling drugs, diuretics and platelet agglutination inhibitors, anticoagulants, lipid-lowering drugs, Cordyceps formulations, and other kidney-protecting and detoxifying drugs.

IgAN is the most common primary glomerular disease worldwide and pathologically manifests itself in localized mesangial hyperplasia and increases in the matrix accompanied by diffuse mesangial deposition of the IgA protein and often by IgG, C3, and C5b-9 deposition. The complement pathways are therefore thought to correlate with the development and progression of IgAN. Currently, there are two small-molecule drugs targeted at the complement pathways that are undergoing clinical trials. OMS721 is a humanized monoclonal antibody targeting the MASP-2 protein developed by Omeros Inc. The MASP-2 protein is the effector enzyme that activates the lectin pathway of the complement system. At the end of the phase 2 clinical trials of OMS721, the 4 IgAN patients enrolled in the trial all had a significantly improved proteinuria index. The drug is currently undergoing phase three clinical trials.

Patent applications that disclose factor B inhibitors include WO2015009616A1, WO2019043609A1, WO2020016749A2, and the like. Application No. WO2022143845 provides a range of nitrogen-containing heterocyclic derivatives, including 4-((1S,3S,5R)-3-ethoxy-8-((5-methoxy-7-methyl-1H-indol-4-yl)methyl)-8-azabicyclo[3.2.1]octan-1-yl)benzoic acid, and they are structurally characterized. Additionally, 4-((1S,3S,5R)-3-ethoxy-8-((5-methoxy-7-methyl-1H-indol-4-yl)methyl)-8-azabicyclo[3.2.1]octan-1-yl)benzoic acid (compound I) was biologically evaluated in the application, and the results show that the compound has a relatively good inhibitory effect on the enzyme activity of Factor B.

The structure of a crystal form of a pharmaceutical active ingredient generally affects the chemical stability of the drug, and the differences in crystallization conditions and storage conditions may cause changes in the structure of the crystal form of the compound and sometimes generation of other crystal forms. Generally, amorphous drug products have no regular crystal form structure and often have other defects, such as poor product stability, fine powder, difficulty in filtration, ease of agglomeration, and poor flowability. Therefore, it is necessary to improve various properties of the above products, and intensive research is needed to find crystal forms with relatively high crystal form purity and good physicochemical stability.

### SUMMARY

The present disclosure provides a salt of a Factor B inhibitor, a crystal form of the salt, a preparation method therefor, and use thereof.

The present disclosure provides a pharmaceutically acceptable salt of compound I, which is a Factor B inhibitor with the chemical name 4-((1S,3S,5R)-3-ethoxy-8-((5-methoxy-7-methyl-1H-indol-4-yl)methyl)-8-azabicyclo[3.2.1]octan-1-yl)benzoic acid. The pharmaceutically acceptable salt is selected from the group consisting of a maleate, a phosphate, a p-toluenesulfonate, a sulfate, a hydrochloride, a fumarate, a tartrate, a succinate, a citrate, a malate, a mesylate, and a hydrobromide. In some embodiments, the pharmaceutically acceptable salt of compound I is 4-((1S,3S,5R)-3-ethoxy-8-((5-methoxy-7-methyl-1H-indol-4-yl)methyl)-8-azabicyclo[3.2.1]octan-1-yl)benzoic acid-fumarate.

In some embodiments, the pharmaceutically acceptable salt of compound I is 4-((1S,3S,5R)-3-ethoxy-8-((5-methoxy-7-methyl-1H-indol-4-yl)methyl)-8-azabicyclo[3.2.1]octan-1-yl)benzoic acid-p-toluenesulfonate.

In some embodiments, the pharmaceutically acceptable salt of compound I is 4-((1S,3S,5R)-3-ethoxy-8-((5-methoxy-7-methyl-1H-indol-4-yl)methyl)-8-azabicyclo[3.2.1]octan-1-yl)benzoic acid-hydrochloride.

In some embodiments, the pharmaceutically acceptable salt of compound I is 4-((1S,3S,5R)-3-ethoxy-8-((5-methoxy-7-methyl-1H-indol-4-yl)methyl)-8-azabicyclo[3.2.1]octan-1-yl)benzoic acid-phosphate.

The present disclosure provides a preparation method for a pharmaceutically acceptable salt of compound I, comprising a step of reacting 4-((1S,3S,5R)-3-ethoxy-8-((5-methoxy-7-methyl-1H-indol-4-yl)methyl)-8-azabicyclo[3.2.1]octan-1-yl)benzoic acid with an acid, wherein the acid is selected from the group consisting of maleic acid, phosphoric acid, p-toluenesulfonic acid, sulfuric acid, hydrochloric acid, fumaric acid, tartaric acid, succinic acid, citric acid, malic acid, methanesulfonic acid, and hydrobromic acid.

In some embodiments, the present disclosure provides a crystal form I of the maleate of compound I, and an X-ray powder diffraction pattern, expressed in terms of 2θ angles, which are diffraction angles, of the crystal form has characteristic peaks at 6.7, 7.6, 8.6, 11.0, 12.1, and 16.2, optionally at 6.7, 7.6, 8.1, 8.6, 11.0, 12.1, 16.2, 19.7, and 23.5, and optionally at 6.7, 7.6, 8.1, 8.6, 9.3, 11.0, 12.1, 13.5, 16.2, 17.9, 19.7, and 23.5.

In some embodiments, the present disclosure provides a crystal form I of the phosphate of compound I, and an X-ray powder diffraction pattern, expressed in terms of 2θ angles, which are diffraction angles, of the crystal form has characteristic peaks at 8.4, 10.3, 11.7, 14.8, 19.2, and 21.8, optionally at 8.4, 10.3, 11.7, 12.5, 14.8, 19.2, 19.8, 21.8, and 23.9, and optionally at 7.0, 8.4, 9.3, 10.3, 11.7, 12.5, 14.8, 17.4, 19.2, 19.8, 21.8, and 23.9.

In some embodiments, the present disclosure provides a crystal form II of the phosphate of compound I, and an X-ray powder diffraction pattern, expressed in terms of 2θ angles, which are diffraction angles, of the crystal form has characteristic peaks at 8.4, 9.5, 10.2, 11.7, 14.7, and 19.1, optionally at 6.9, 8.4, 9.5, 10.2, 10.7, 11.7, 14.7, 18.5, and 19.1, and optionally at 6.9, 8.4, 8.8, 9.5, 10.2, 10.7, 11.7, 14.7, 15.7, 18.5, 19.1, and 19.8.

In some embodiments, the present disclosure provides a crystal form III of the phosphate of compound I, and an X-ray powder diffraction pattern, expressed in terms of 2θ angles, which are diffraction angles, of the crystal form has characteristic peaks at 7.0, 8.0, 9.8, 11.5, 18.5, and 21.3, optionally at 7.0, 8.0, 9.8, 11.5, 16.1, 18.0, 18.5, 21.3, and 24.1, and optionally at 7.0, 8.0, 9.8, 11.5, 16.1, 18.0, 18.5, 20.8, 21.3, 22.9, 24.1, and 25.3.

In some embodiments, the present disclosure provides a crystal form IV of the phosphate of compound I, and an X-ray powder diffraction pattern, expressed in terms of 2θ angles, which are diffraction angles, of the crystal form has characteristic peaks at 6.9, 8.4, 10.3, 11.7, and 14.8.

In some embodiments, the present disclosure provides a crystal form V of the phosphate of compound I, and an X-ray powder diffraction pattern, expressed in terms of 2θ angles, which are diffraction angles, of the crystal form has characteristic peaks at 9.1, 10.2, 11.5, 15.7, and 19.8, optionally at 8.6, 9.1, 10.2, 11.5, 15.7, 18.0, 19.8, and 23.5.

In some embodiments, the present disclosure provides a crystal form I of the p-toluenesulfonate of compound I, and an X-ray powder diffraction pattern, expressed in terms of 2θ angles, which are diffraction angles, of the crystal form has characteristic peaks at 5.0, 9.4, 10.1, 16.3, and 18.3, optionally at 5.0, 9.4, 10.1, 16.3, 18.3, 18.9, 21.2, and 22.9, and optionally at 5.0, 9.4, 10.1, 16.0, 16.3, 17.1, 18.3, 18.9, 21.2, 22.9, and 24.0.

In some embodiments, the present disclosure provides a crystal form II of the p-toluenesulfonate of compound I, and an X-ray powder diffraction pattern, expressed in terms of 2θ angles, which are diffraction angles, of the crystal form has characteristic peaks at 4.7, 8.8, 9.3, 10.8, 13.9, and 18.7, optionally at 4.7, 8.8, 9.3, 9.7, 10.8, 13.9, 17.7, and 18.7.

In some embodiments, the present disclosure provides a crystal form III of the p-toluenesulfonate of compound I, and an X-ray powder diffraction pattern, expressed in terms of 2θ angles, which are diffraction angles, of the crystal form has characteristic peaks at 6.8, 7.4, 8.1, 10.1, and 12.7.

In some embodiments, the present disclosure provides a crystal form I of the sulfate of compound I, and an X-ray powder diffraction pattern, expressed in terms of 2θ angles, which are diffraction angles, of the crystal form has characteristic peaks at 7.2, 9.2, 17.1, 20.0, 21.4, and 24.7, optionally at 6.7, 7.2, 9.2, 17.1, 18.7, 20.0, 21.4, 22.9, and 24.7.

In some embodiments, the present disclosure provides a crystal form II of the sulfate of compound I, and an X-ray powder diffraction pattern, expressed in terms of 2θ angles, which are diffraction angles, of the crystal form has characteristic peaks at 9.5, 10.2, 16.6, 21.2, and 25.7, optionally at 6.3, 8.5, 9.5, 10.2, 16.6, 19.8, 21.2, 23.7, and 25.7.

In some embodiments, the present disclosure provides a crystal form III of the sulfate of compound I, and an X-ray powder diffraction pattern, expressed in terms of 2θ angles, which are diffraction angles, of the crystal form has characteristic peaks at 6.9, 7.6, 9.1, 18.2, and 23.7, optionally at 6.9, 7.6, 9.1, 17.0, 18.2, 20.7, 23.7, and 24.0.

In some embodiments, the present disclosure provides a crystal form IV of the sulfate of compound I, and an X-ray powder diffraction pattern, expressed in terms of 2θ angles, which are diffraction angles, of the crystal form has characteristic peaks at 6.9, 12.5, 16.5, 19.4, 21.2, and 24.0, optionally at 6.9, 9.7, 12.5, 16.5, 19.4, 21.2, 24.0, and 25.8.

In some embodiments, the present disclosure provides a crystal form V of the sulfate of compound I, and an X-ray powder diffraction pattern, expressed in terms of 2θ angles, which are diffraction angles, of the crystal form has characteristic peaks at 7.6, 11.4, 13.5, 17.2, 18.8, and 19.5, optionally at 7.6, 10.0, 11.4, 13.5, 14.0, 17.2, 19.5, 22.5, and 24.6.

In some embodiments, the present disclosure provides a crystal form VI of the sulfate of compound I, and an X-ray powder diffraction pattern, expressed in terms of 2θ angles, which are diffraction angles, of the crystal form has characteristic peaks at 6.7, 8.8, 14.6, 15.9, and 23.7, optionally at 6.7, 8.8, 10.6, 14.6, 15.9, 19.5, 21.4, and 23.7.

In some embodiments, the present disclosure provides a crystal form I of the hydrochloride of compound I, and an X-ray powder diffraction pattern, expressed in terms of 2θ angles, which are diffraction angles, of the crystal form has characteristic peaks at 5.8, 8.8, 11.6, 20.7, and 23.4, optionally at 5.8, 8.8, 9.8, 10.5, 11.6, 14.6, 18.4, 20.7, and 23.4.

In some embodiments, the present disclosure provides a crystal form II of the hydrochloride of compound I, and an X-ray powder diffraction pattern, expressed in terms of 2θ angles, which are diffraction angles, of the crystal form has characteristic peaks at 5.9, 8.8, 10.6, 17.2, 19.3, and 23.9, optionally at 5.9, 8.8, 10.6, 13.2, 17.2, 19.3, 21.3, 23.9, 24.4, and 26.1, and optionally at 5.9, 8.8, 10.6, 11.9, 13.2, 14.7, 17.2, 19.3, 19.9, 21.3, 23.9, 24.4, 26.1, and 27.4.

In some embodiments, the present disclosure provides a crystal form III of the hydrochloride of compound I, and an X-ray powder diffraction pattern, expressed in terms of 2θ angles, which are diffraction angles, of the crystal form has characteristic peaks at 6.1, 8.8, 10.4, 18.4, 19.9, and 24.6, optionally at 6.1, 8.8, 10.4, 12.2, 18.4, 19.9, 22.6, 24.6, and 28.0, and optionally at 6.1, 8.8, 10.4, 12.2, 14.6, 16.6, 17.8, 18.4, 19.9, 22.6, 24.6, 27.2, and 28.0.

In some embodiments, the present disclosure provides a crystal form IV of the hydrochloride of compound I, and an X-ray powder diffraction pattern, expressed in terms of 2θ angles, which are diffraction angles, of the crystal form has characteristic peaks at 5.4, 9.0, 10.8, 20.4, and 21.8, optionally at 5.4, 9.0, 10.8, 19.3, 20.4, 21.8, and 27.3. In some embodiments, the present disclosure provides a crystal form V of the hydrochloride of compound I, and an X-ray powder diffraction pattern, expressed in terms of 2θ angles, which are diffraction angles, of the crystal form has characteristic peaks at 5.2, 6.7, 7.7, 10.2, and 17.4, optionally at 5.2, 6.7, 7.7, 10.2, 10.8, 17.4, 20.5, and 24.2.

In some embodiments, the present disclosure provides a crystal form VI of the hydrochloride of compound I, and an X-ray powder diffraction pattern, expressed in terms of 2θ angles, which are diffraction angles, of the crystal form has characteristic peaks at 5.8, 10.3, 11.7, 17.7, 20.7, and 23.7. In some embodiments, the present disclosure provides a crystal form I of the fumarate of compound I, and an X-ray powder diffraction pattern, expressed in terms of 2θ angles, which are diffraction angles, of the crystal form has characteristic peaks at 9.6, 14.0, 16.7, 19.6, 25.8, and 26.1, optionally at 6.1, 9.6, 10.0, 14.0, 16.7, 17.2, 19.1, 19.6, 25.8, and 26.1, and optionally at 6.1, 9.6, 10.0, 10.8, 14.0, 16.7, 17.2, 18.6, 19.1, 19.6, 20.2, 25.8, and 26.1.

In some embodiments, the present disclosure provides a crystal form II of the fumarate of compound I, and an X-ray powder diffraction pattern, expressed in terms of 2θ angles, which are diffraction angles, of the crystal form has characteristic peaks at 6.2, 6.6, 8.0, 13.2, 14.0, 20.3, and 24.2, optionally at 6.2, 6.6, 8.0, 9.0, 13.2, 14.0, 16.4, 17.1, 19.8, 20.3, 24.2, and 25.3, and optionally at 6.2, 6.6, 8.0, 9.0, 12.0, 13.2, 14.0, 16.4, 17.1, 19.3, 19.8, 20.3, 21.9, 22.3, 24.2, 25.3, 25.7, and 28.1.

In some embodiments, the present disclosure provides a crystal form I of the hydrobromide of compound I, and an X-ray powder diffraction pattern, expressed in terms of 2θ angles, which are diffraction angles, of the crystal form has characteristic peaks at 7.6, 10.6, 16.4, 18.4, 22.6, and 24.0, optionally at 7.6, 10.6, 15.3, 16.4, 18.4, 19.6, 22.6, 24.0, 26.5, and 27.0, and optionally at 7.6, 10.6, 15.3, 16.4, 18.4, 19.6, 21.4, 22.6, 24.0, 25.5, 26.5, 27.0, and 28.9.

In some embodiments, the present disclosure provides a crystal form II of the hydrobromide of compound I, and an X-ray powder diffraction pattern, expressed in terms of 2θ angles, which are diffraction angles, of the crystal form has characteristic peaks at 7.2, 10.5, 16.5, 22.5, 23.4, and 26.6, optionally at 7.2, 10.5, 13.1, 16.5, 18.8, 20.3, 22.5, 23.4, and 26.6, and optionally at 7.2, 10.5, 13.1, 16.5, 17.2, 18.8, 20.3, 21.5, 21.9, 22.5, 23.4, and 26.6.

In some embodiments, the present disclosure provides an amorphous form of the maleate of compound I, and an X-ray powder diffraction pattern of the amorphous form has no significant characteristic peaks within a range of 2θ diffraction angles of 3-48°.

In some embodiments, the present disclosure provides an amorphous form of the phosphate of compound I, and an X-ray powder diffraction pattern of the amorphous form has no significant characteristic peaks within a range of 2θ diffraction angles of 3-48°.

In some embodiments, the present disclosure provides an amorphous form of the p-toluenesulfonate of compound I, and an X-ray powder diffraction pattern of the amorphous form has no significant characteristic peaks within a range of 2θ diffraction angles of 3-48°.

In some embodiments, the present disclosure provides an amorphous form of the sulfate of compound I, and an X-ray powder diffraction pattern of the amorphous form has no significant characteristic peaks within a range of 2θ diffraction angles of 3-48°.

In some embodiments, the present disclosure provides an amorphous form of the tartrate of compound I, and an X-ray powder diffraction pattern of the amorphous form has no significant characteristic peaks within a range of 2θ diffraction angles of 3-48°.

In some embodiments, the present disclosure provides an amorphous form of the succinate of compound I, and an X-ray powder diffraction pattern of the amorphous form has no significant characteristic peaks within a range of 2θ diffraction angles of 3-48°.

In some embodiments, the present disclosure provides an amorphous form of the fumarate of compound I, and an X-ray powder diffraction pattern of the amorphous form has no significant characteristic peaks within a range of 2θ diffraction angles of 3-48°.

In some embodiments, the present disclosure provides an amorphous form of the citrate of compound I, and an X-ray powder diffraction pattern of the amorphous form has no significant characteristic peaks within a range of 2θ diffraction angles of 3-48°.

In some embodiments, the present disclosure provides an amorphous form of the malate of compound I, and an X-ray powder diffraction pattern of the amorphous form has no significant characteristic peaks within a range of 2θ diffraction angles of 3-48°.

In some embodiments, the present disclosure provides an amorphous form of the hydrobromide of compound I, and an X-ray powder diffraction pattern of the amorphous form has no significant characteristic peaks within a range of 2θ diffraction angles of 3-48°.

In some embodiments, the present disclosure provides an amorphous form of the mesylate of compound I, and an X-ray powder diffraction pattern of the amorphous form has no significant characteristic peaks within a range of 2θ diffraction angles of 3-48°.

In some embodiments, the present disclosure provides an amorphous form of the hydrochloride of compound I, and an X-ray powder diffraction pattern of the amorphous form has no significant characteristic peaks within a range of 2θ diffraction angles of 3-48°.

In an optional embodiment, for the crystal forms of the pharmaceutically acceptable salts of compound I provided by the present disclosure, the 2θ angles have a margin of error of ±0.2.

In another aspect, the present disclosure provides a preparation method for the crystal form I of the maleate of compound I, comprising: a. dissolving compound I in acetonitrile, adding a maleic acid solution, and slurrying, and b. adding isopropyl ether and crystallizing.

The present disclosure provides a preparation method for the crystal form I of the phosphate of compound I, comprising adding compound I to a solvent (1) and phosphoric acid and stirring for crystallization, wherein the solvent (1) is selected from the group consisting of acetonitrile and acetone.

The present disclosure provides a preparation method for the crystal form II of the phosphate of compound I, comprising adding compound I to a solvent (2) and phosphoric acid and stirring for crystallization, wherein the solvent (2) is selected from the group consisting of ethyl acetate and acetone.

The present disclosure provides a preparation method for the crystal form III of the phosphate of compound I, comprising adding compound I to a solvent (3) and phosphoric acid and stirring for crystallization, wherein the solvent (3) is selected from the group consisting of isopropanol and ethanol.

The present disclosure provides a preparation method for the crystal form IV of the phosphate of compound I, comprising adding compound I to acetonitrile and phosphoric acid and stirring for crystallization.

The present disclosure provides a preparation method for the crystal form V of the phosphate of compound I, comprising adding compound I to ethanol and phosphoric acid and stirring for crystallization.

The present disclosure provides a preparation method for the crystal form I of the p-toluenesulfonate of compound I, including method 1: adding compound I to a solvent (4) and p-toluenesulfonic acid, slurrying at room temperature, adding isopropyl ether, and stirring for crystallization, wherein the solvent (4) is selected from the group consisting of ethanol, isopropanol, and ethyl acetate; and method 2: adding compound I to acetonitrile and p-toluenesulfonic acid and stirring for crystallization.

The present disclosure provides a preparation method for the crystal form II of the p-toluenesulfonate of compound I, comprising adding compound I to isopropanol and p-toluenesulfonic acid and stirring for crystallization.

The present disclosure provides a preparation method for the crystal form III of the p-toluenesulfonate of compound I, comprising adding the crystal form II of the p-toluenesulfonate to methyl tert-butyl ether and stirring for crystallization.

The present disclosure provides a preparation method for the crystal form I of the sulfate of compound I, comprising adding compound I to a solvent (5) and sulfuric acid and stirring for crystallization, wherein the solvent (5) is selected from the group consisting of ethanol and acetonitrile.

The present disclosure provides a preparation method for the crystal form II of the sulfate of compound I, comprising adding compound I to acetone and sulfuric acid and stirring for crystallization.

The present disclosure provides a preparation method for the crystal form III of the sulfate of compound I, comprising dissolving compound I in ethanol, adding sulfuric acid, adding isopropyl ether, and stirring for crystallization.

The present disclosure provides a preparation method for the crystal form IV of the sulfate of compound I, comprising adding compound I to a solvent (6) and sulfuric acid and stirring for crystallization, wherein the solvent (6) is selected from the group consisting of isopropanol and acetone.

The present disclosure provides a preparation method for the crystal form V of the sulfate of compound I, comprising adding compound I to a solvent (7) and sulfuric acid and stirring for crystallization, wherein the solvent (7) is selected from the group consisting of ethyl acetate and isopropyl acetate.

The present disclosure provides a preparation method for the crystal form VI of the sulfate of compound I, comprising adding the crystal form I of the sulfate to isopropyl acetate and stirring for crystallization.

The present disclosure provides a preparation method for the crystal form I of the hydrochloride of compound I, including method 1: dissolving compound I in ethanol and hydrochloric acid, adding isopropyl ether, and stirring for crystallization; and method 2: adding compound I to isopropyl acetate and hydrochloric acid and stirring for crystallization.

The present disclosure provides a preparation method for the crystal form II of the hydrochloride of compound I, including method 1: dissolving compound I in ethanol, adding hydrochloric acid, adding isopropyl ether, and stirring for crystallization; and method 2: adding compound I to a solvent (8) and hydrochloric acid and stirring for crystallization, wherein the solvent (8) is selected from the group consisting of isopropanol and acetonitrile.

The present disclosure provides a preparation method for the crystal form III of the hydrochloride of compound I, comprising adding compound I to a solvent (9) and hydrochloric acid and stirring for crystallization, wherein the solvent (9) is selected from the group consisting of acetone and ethyl acetate.

The present disclosure provides a preparation method for the crystal form IV of the hydrochloride of compound I, comprising adding compound I to a solvent (10) and hydrochloric acid and stirring for crystallization, wherein the solvent (10) is selected from the group consisting of tetrahydrofuran and isopropanol.

The present disclosure provides a preparation method for the crystal form V of the hydrochloride of compound I, comprising adding compound I to acetonitrile and hydrochloric acid and stirring for crystallization.

The present disclosure provides a preparation method for the crystal form VI of the hydrochloride of compound I, comprising adding compound I to isopropanol and hydrochloric acid and stirring for crystallization.

The present disclosure provides a preparation method for the crystal form I of the fumarate of compound I, comprising adding compound I to a solvent (11) and fumaric acid and stirring for crystallization, wherein the solvent (11) is selected from the group consisting of acetonitrile and acetone.

The present disclosure provides a preparation method for the crystal form II of the fumarate of compound I, comprising adding compound I to a methanol/acetonitrile solution and fumaric acid and volatilizing for crystallization.

The present disclosure provides a preparation method for the crystal form I of the hydrobromide of compound I, comprising dissolving compound I in ethanol, adding hydrobromic acid, adding isopropyl ether, and stirring for crystallization.

The present disclosure provides a preparation method for the crystal form II of the hydrobromide of compound I, comprising adding compound I to a solvent (12) and hydrobromic acid and stirring for crystallization, wherein the solvent (12) is selected from the group consisting of isopropanol and ethyl acetate.

The present disclosure provides a preparation method for the amorphous form of the maleate of compound I, comprising dissolving compound I in a solvent (13) and maleic acid, then adding isopropyl ether, and stirring for crystallization, wherein the solvent (13) is selected from the group consisting of at least one of ethanol, acetone, tetrahydrofuran and acetonitrile/methanol, isopropanol, and ethyl acetate.

The present disclosure provides a preparation method for the amorphous form of the phosphate of compound I, including method 1: dissolving compound I in acetonitrile/methanol and phosphoric acid, slurrying at room temperature, then adding isopropyl ether, and stirring for crystallization; and method 2: adding compound I to a solvent (14) and phosphoric acid and stirring for crystallization, wherein the solvent (14) is selected from the group consisting of ethanol and tetrahydrofuran.

The present disclosure provides a preparation method for the morphous p-toluenesulfonate of compound I, comprising adding compound I to a solvent (15) and p-toluenesulfonic acid, slurrying at room temperature, then adding isopropyl ether, and stirring for crystallization, wherein the solvent (15) is selected from the group consisting of at least one of acetonitrile, acetone, tetrahydrofuran, and acetonitrile/methanol.

The present disclosure provides a preparation method for the amorphous form of the sulfate of compound I, including method 1: dissolving compound I in acetonitrile/methanol, adding sulfuric acid, slurrying at room temperature, then adding isopropyl ether, and crystallizing; and method 2: adding compound I to tetrahydrofuran and sulfuric acid and crystallizing.

The present disclosure provides a preparation method for the amorphous form of the tartrate of compound I, including method 1: dissolving compound I in a solvent (16), adding tartaric acid, slurrying at room temperature, then adding isopropyl ether, and crystallizing, wherein the solvent (16) is selected from the group consisting of ethanol and tetrahydrofuran; and method 2: adding compound I to a solvent (17) and tartaric acid and crystallizing, wherein the solvent (17) is selected from the group consisting of acetonitrile and acetone.

The present disclosure provides a preparation method for the amorphous form of the succinate of compound I, including method 1: dissolving compound I in a solvent (18), adding succinic acid, slurrying at room temperature, then adding isopropyl ether, and crystallizing, wherein the solvent (18) is selected from the group consisting of ethanol and tetrahydrofuran; and method 2: adding compound I to a solvent (19) and succinic acid and crystallizing, wherein the solvent (19) is selected from the group consisting of acetonitrile and acetone.

The present disclosure provides a preparation method for the amorphous form of the fumarate of compound I, including method 1: dissolving compound I in a solvent (20), adding fumaric acid, slurrying at room temperature, then adding isopropyl ether, and crystallizing, wherein the solvent (20) is selected from the group consisting of ethanol and tetrahydrofuran; and method 2: adding compound I to acetone and fumaric acid and crystallizing.

The present disclosure provides a preparation method for the amorphous form of the citrate of compound I, comprising dissolving compound I in a solvent (21), adding citric acid, slurrying at room temperature, then adding isopropyl ether, and crystallizing, wherein the solvent (21) is selected from the group consisting of at least one of ethanol, acetonitrile, acetone, and tetrahydrofuran.

The present disclosure provides a preparation method for the amorphous form of the malate of compound I, including method 1: dissolving compound I in a solvent (22), adding malic acid, slurrying at room temperature, then adding isopropyl ether, and crystallizing, wherein the solvent (22) is selected from the group consisting of at least one of ethanol, acetone, and tetrahydrofuran; and method 2: adding compound I to acetonitrile and malic acid and crystallizing.

The present disclosure provides a preparation method for the amorphous form of the hydrobromide of compound I, comprising dissolving compound I in a solvent (23) and acetonitrile, adding hydrobromic acid, slurrying at room temperature, then adding isopropyl ether, and crystallizing, wherein the solvent (23) is selected from the group consisting of at least one of acetonitrile, acetone, and tetrahydrofuran.

The present disclosure provides a preparation method for the amorphous form of the mesylate of compound I, comprising dissolving compound I in a solvent (24), adding methanesulfonic acid, slurrying at room temperature, then adding isopropyl ether, and crystallizing, wherein the solvent (24) is selected from the group consisting of at least one of acetonitrile, acetone, tetrahydrofuran, and ethanol.

The present disclosure provides a preparation method for the amorphous form of the hydrochloride of compound I, comprising dissolving compound I in ethanol, adding hydrochloric acid, slurrying at room temperature, then adding isopropyl ether, and crystallizing.

In certain embodiments, the preparation methods for the crystal forms described in the present disclosure also comprise a filtration, washing, or drying step.

The present disclosure also provides a pharmaceutical composition prepared from an aforementioned crystal form of a pharmaceutically acceptable salt of compound I.

The present disclosure also provides a pharmaceutical composition comprising an aforementioned pharmaceutically acceptable salt of compound I, a crystal form of the pharmaceutically acceptable salt, or a mixture thereof, or a pharmaceutically acceptable salt of compound I or a crystal form of the pharmaceutically acceptable salt prepared by an aforementioned method, and optionally a pharmaceutically acceptable excipient.

The present disclosure also provides a preparation method for a pharmaceutical composition, comprising a step of mixing an aforementioned pharmaceutically acceptable salt of compound I, a crystal form of the pharmaceutically acceptable salt, or a mixture thereof, or a pharmaceutically acceptable salt of compound I or a crystal form of the pharmaceutically acceptable salt prepared by an aforementioned method, with a pharmaceutically acceptable excipient.

The present disclosure also provides use of a pharmaceutically acceptable salt of compound I, a crystal form of the pharmaceutically acceptable salt, or a mixture thereof, or a pharmaceutically acceptable salt or a crystal form of the pharmaceutically acceptable salt prepared by an aforementioned method or a mixture thereof, or the aforementioned composition, or a composition prepared by the aforementioned method in the preparation of a medicament for inhibiting the activation of the complement alternative pathway.

The present disclosure also provides use of a pharmaceutically acceptable salt of compound I, a crystal form of the pharmaceutically acceptable salt, or a mixture thereof, or a pharmaceutically acceptable salt or a crystal form of the pharmaceutically acceptable salt prepared by an aforementioned method or a mixture thereof, or the aforementioned composition in the preparation of a medicament for treating a disease or disorder, wherein the disease or disorder is selected from the group consisting of glomerulopathy, hemolytic uremic syndrome, atypical haemolytic uraemic syndrome, paroxysmal nocturnal hemoglobinuria, age-related macular degeneration, geographic atrophy, diabetic retinopathy, uveitis, retinitis pigmentosa, macular edema, Behcet's uveitis, multifocal choroiditis, Vogt-Koyanagi-Harada syndrome, birdshot retino-chorioditis, sympathetic ophthalmia, ocular dicatricial pemphigoid, ocular pemphigus, nonartertic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious disease or sepsis, systemic lupus erythematosus, systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome, asthma, chronic obstructive pulmonary disease, emphysema, pulmonary embolisms and infarcts, pneumonia, fibrogenic dust diseases, pulmonary fibrosis, asthma, allergy, bronchoconstriction, parasitic diseases, Goodpasture's syndrome, pulmonary vasculitis, pauci-immune vasculitis, immune complex-associated inflammation, antiphospholipid syndrome, and obesity; the disease or disorder is preferably C3 glomerulopathy, IgA nephropathy, membranous glomerulonephritis, atypical haemolytic uraemic syndrome, and paroxysmal nocturnal hemoglobinuria.

As used herein, "2θ or 2θ angle" refers to a diffraction angle; θ refers to the Bragg angle in ° or degrees; the 2θ of each characteristic peak has a margin of error of ±0.20 (including the case that a number having more than 1 decimal place is rounded), specifically -0.20, -0.19, -0.18, -0.17, -0.16, - 0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

As used herein, "crystallization" or "crystallizing" includes, but is not limited to, stirring crystallization, triturating crystallization, cooling crystallization, and volatilizing crystallization.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain the phase change information about the sample.

The drying described herein is generally performed at a temperature of 25 °C-100 °C, preferably 40 °C-70 °C, and may be performed under atmospheric pressure or reduced pressure.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the crystal form I of the maleate of compound I.
FIG. 2 shows an XRPD pattern of the crystal form I of the phosphate of compound I.
FIG. 3 shows an XRPD pattern of the crystal form II of the phosphate of compound I.
FIG. 4 shows an XRPD pattern of the crystal form III of the phosphate of compound I.
FIG. 5 shows an XRPD pattern of the crystal form IV of the phosphate of compound I.
FIG. 6 shows an XRPD pattern of the crystal form V of the phosphate of compound I.
FIG. 7 shows an XRPD pattern of the crystal form I of the p-toluenesulfonate of compound I.
FIG. 8 shows an XRPD pattern of the crystal form II of the p-toluenesulfonate of compound I.
FIG. 9 shows an XRPD pattern of the crystal form III of the p-toluenesulfonate of compound I.
FIG. 10 shows an XRPD pattern of the crystal form I of the sulfate of compound I.
FIG. 11 shows an XRPD pattern of the crystal form II of the sulfate of compound I.
FIG. 12 shows an XRPD pattern of the crystal form III of the sulfate of compound I.
FIG. 13 shows an XRPD pattern of the crystal form IV of the sulfate of compound I.
FIG. 14 shows an XRPD pattern of the crystal form V of the sulfate of compound I.
FIG. 15 shows an XRPD pattern of the crystal form VI of the sulfate of compound I.
FIG. 16 shows an XRPD pattern of the crystal form I of the hydrochloride of compound I.
FIG. 17 shows an XRPD pattern of the crystal form II of the hydrochloride of compound I.
FIG. 18 shows an XRPD pattern of the crystal form III of the hydrochloride of compound I.
FIG. 19 shows an XRPD pattern of the crystal form IV of the hydrochloride of compound I.
FIG. 20 shows an XRPD pattern of the crystal form V of the hydrochloride of compound I.
FIG. 21 shows an XRPD pattern of the crystal form VI of the hydrochloride of compound I.
FIG. 22 shows an XRPD pattern of the crystal form I of the fumarate of compound I.
FIG. 23 shows an XRPD pattern of the crystal form II of the fumarate of compound I.
FIG. 24 shows an XRPD pattern of the crystal form I of the hydrobromide of compound I.
FIG. 25 shows an XRPD pattern of the crystal form II of the hydrobromide of compound I.
FIG. 26 shows an XRPD pattern of the amorphous form of the fumarate of compound I.

### DETAILED DESCRIPTION

The present disclosure is further illustrated in detail by the following examples and experimental examples. These examples and experimental examples are for illustrative purposes only and are not intended to limit the scope of the present disclosure.

Test conditions for the instruments used in the experiment: The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-d6), chloroform-D (CDCl3), and methanol-D4 (CD3OD) as solvents, and tetramethylsilane (TMS) as an internal standard.

The MS analyses were performed using a FINNIGAN LCQAd (ESI) mass spectrometer (manufacturer: Thermo, model: Finnigan LCQ advantage MAX).

The HPLC analyses were performed using an Agilent 1260DAD high pressure liquid chromatograph (Sunfire C18 150 × 4.6 mm chromatography column) and a Thermo U3000 high pressure liquid chromatograph (Gimini C18 150 × 4.6 mm chromatography column).

XRPD refers to X-ray powder diffraction: The measurement was performed using a BRUKER D8 X-ray diffractometer, and the specific acquisition information was: a Cu anode (40 kV, 40 mA), radiation: monochromatic Cu-Ka radiation (l = 1.5418 Å). Scan mode: θ/2θ, scan range: 3-48°. DSC refers to differential scanning calorimetry: The measurement was performed using a METTLER TOLEDO DSC 3+ differential scanning calorimeter, with a temperature ramp rate of 10 °C/min, 25-350 °C, and a nitrogen purge speed of 50 mL/min.

TGA refers to thermogravimetric analysis: The measurement was performed using a METTLER TOLEDO TGA 2 thermogravimetric analyzer, with a temperature ramp rate of 10 °C/min, specific temperature ranges shown in corresponding patterns, and a nitrogen purge speed of 50 mL/min.

DVS refers to dynamic vapor sorption: Surface Measurement Systems instrinsic was adopted; the humidity started at 50%, the humidity range investigated was 0%-95%, and the humidity was increased in increments of 10%; the criterion was that each gradient mass change dM/dT is less than 0.002%, TMAX 360 min, two cycles.

### Example 1. Preparation of Compound I (with reference to the preparation methods of Examples 1-2 in Application No. PCT/CN2021/142760)

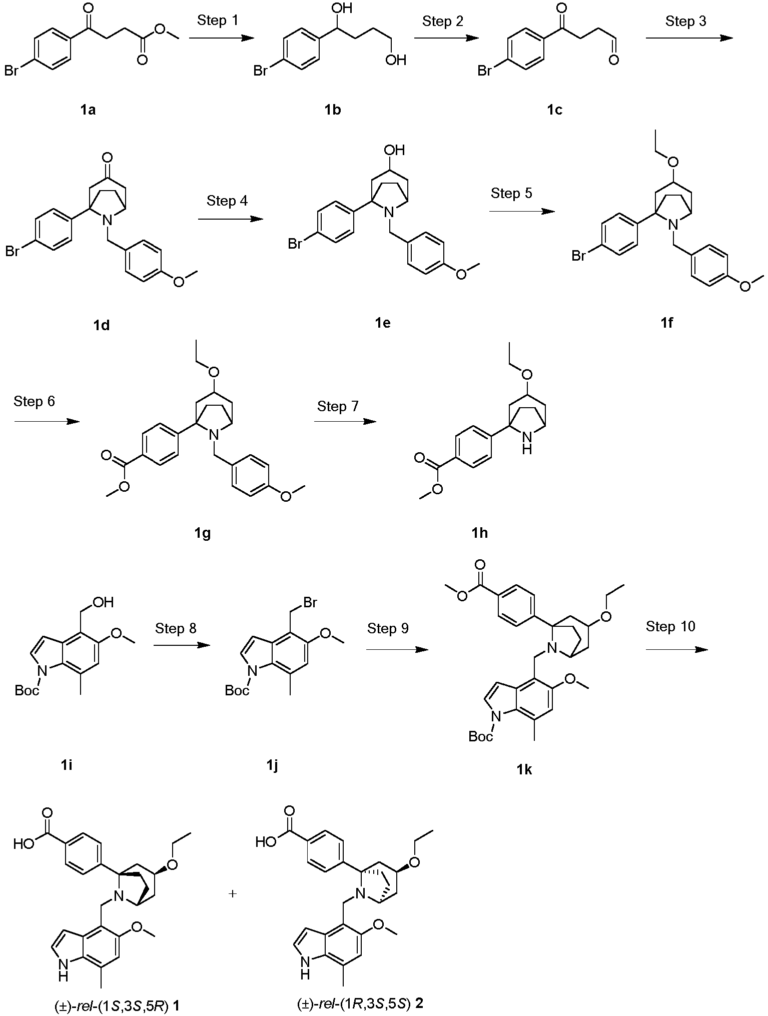

### Step 1

### 1-(4-Bromophenyl)butane-1,4-diol 1b

Methyl 4-(4-bromophenyl)-4-oxobutanoate **1a** (5 g, 17.54 mmol, Bide Pharmatech Ltd.) was dissolved in tetrahydrofuran (50 mL), and a solution of lithium borohydride in tetrahydrofuran (17 mL, 2 mmol/mL) was added at 0 °C. The mixture was naturally warmed to room temperature and stirred overnight. The reaction mixture was quenched with a saturated sodium thiosulfate solution and extracted with ethyl acetate. The organic phase was dried and concentrated to give a crude product of the title product **1b** (4.29 g), and the crude product was directly used in the next step without purification.

MS m/z (ESI): 242.9 [M-H].

### Step 2

### 4-(4-Bromophenyl)-4-oxobutanal 1c

Dimethyl sulfoxide (8.2 g, 104.95 mmol) was dissolved in dichloromethane (50 mL), and oxalyl chloride (8.8 g, 69.33 mmol) was added at -78 °C. The mixture was stirred for another 10 min, and compound **1b** (4.29 g, 17.50 mmol) was added. After 10 min, triethylamine (17.7 g, 174.92 mmol) was added. The reaction mixture was stirred for another hour, naturally warmed to room temperature, and diluted with dichloromethane. The organic phase was washed with saturated aqueous sodium bicarbonate solution, dried, concentrated under reduced pressure, and purified by silica gel column chromatography with eluent C to give the title compound **1c** (2.7g, yield: 64%).

MS m/z (ESI): 240.8 [M+1].

### Step 3

### 1-(4-Bromophenyl)-8-[(4-methoxybenzyl)-8-azabicyclo[3.2.1]octan-3-one 1d

4-Methoxybenzylamine (1.61 g, 11.74 mmol, Accela ChemBio Inc.) and sodium acetate (6.43 g, 78.38 mmol) were dissolved in water (7.5 mL), and 2 M hydrochloric acid (16 mL) and 1,3-acetonedicarboxylic acid (1.96 g, 13.42 mmol) were added at 0 °C. The mixture was stirred for another 30 min, and compound **1c** (2.7 g, 11.20 mmol) was added. After 30 min, the mixture was stirred at 40 °C for 3 h. The pH of the reaction mixture was adjusted to 8-9 with a saturated sodium bicarbonate solution, and the mixture was extracted with ethyl acetate. The organic phase was dried, concentrated under reduced pressure, and purified by silica gel column chromatography with eluent C to give the title compound **1d** (580 mg, yield: 12.9%).

MS m/z (ESI): 399.9 [M+1].

### Step 4

### 1-(4-Bromophenyl)-8-(4-methoxybenzyl)-8-azabicyclo[3.2.1]octan-3-ol 1e

Compound **1d** (530 mg, 1.32 mmol) was dissolved in methanol (5 mL), and sodium borohydride (200 mg, 5.29 mmol) was added. The mixture was stirred at room temperature for 2 h. The reaction mixture was quenched with a saturated aqueous ammonium chloride solution and extracted with ethyl acetate. The organic phase was dried and concentrated under reduced pressure to give a crude product of the title compound **1e** (420 mg, yield: 78.8%).

MS m/z (ESI): 401.8 [M+1].

### Step 5

### 1-(4-Bromophenyl)-3-ethoxy-8-(4-methoxybenzyl)-8-azabicyclo[3.2.1]octane 1f

Compound **1e** was dissolved in dimethylformamide (5 mL), and sodium hydride (83 mg, 2.08 mmol) was added at 0 °C. The reaction mixture was stirred for another hour, and iodoethane (325 mg, 2.09 mmol) was added. The reaction mixture was warmed to room temperature and stirred overnight. The reaction mixture was quenched with saturated aqueous ammonium chloride solution and extracted with ethyl acetate. The organic phase was dried, concentrated under reduced pressure, and purified by silica gel column chromatography with eluent C to give the title compound **1f** (350 mg, yield: 77.9%).

MS m/z (ESI): 429.9[M+1].

### Step 6

### Methyl 4-(3-ethoxy-8-(4-methoxybenzyl)-8-azabicyclo[3.2.1]octan-1-yl)benzoate 1g

Compound **1f** was dissolved in methanol (4 mL) and dimethylformamide (4 mL), and palladium acetate (54 mg, 240.52 µmol), diphenyl phosphoryl azide (100 mg, 242.46 µmol), and triethylamine (822 mg, 8.12 mmol) were added. The system was purged three times with carbon monoxide gas, and the mixture was stirred at 80 °C overnight. The reaction mixture was poured into water and extracted with ethyl acetate. The organic phase was dried, concentrated under reduced pressure, and purified by silica gel column chromatography with eluent C to give the title compound **1g** (225 mg, yield: 67.5%).

MS m/z (ESI): 411.0[M+1].

### Step 7

### Methyl 4-(3-ethoxy-8-azabicyclo[3.2.1]octan-1-yl)benzoate 1h

Compound **1g** (225 mg, 549.43 µmol) was dissolved in ethanol (5 mL), and the hydrogenation catalyst palladium on carbon (40 mg, 375.87 µmol) was added. The system was purged three times with hydrogen gas, and the mixture was stirred at room temperature for 48 h in a hydrogen atmosphere. The reaction mixture was filtered. The organic phase was concentrated under reduced pressure to give a crude product of the title compound **1h** (130 mg), and the product was directly used in the next step without purification.

MS m/z (ESI): 290.0[M+1].

### Step 8

### tert-Butyl 4-(bromomethyl)-5-methoxy-7-methylindole-1-carboxylate 1j

The compound tert-butyl 4-(hydroxymethyl)-5-methoxy-7-methyl-1H-indole-1-carboxylate **1i** (150 mg, 514.86 µmol, synthesized with reference to the preparation method for intermediate 1-10 in WO2015009616A1) was dissolved in dichloromethane (2 mL), and carbon tetrabromide (170 mg, 512.62 µmol) and triphenylphosphine (135 mg, 514.71 µmol) were added under a nitrogen atmosphere. The reaction mixture was stirred at room temperature for 2 h and directly concentrated to give crude compound **1j** (183 mg), and the product was directly used in the next step without purification.

### Step 9

### tert-Butyl 4-((3-ethoxy-1-(4-(methoxycarbonyl)phenyl)-8-azabicyclo[3.2.1]octan-8-yl)methyl)-5-methoxy-7-methyl-1H-indole-1-carboxylate 1k

Compound **1h** (100 mg, 345.5801 µmol) was dissolved in dimethylformamide (2 mL), and sodium hydride (27 mg, 675.07 µmol) was added at 0 °C. The reaction mixture was stirred for another hour, and then a solution of compound **1j** (183 mg, 516.60 µmol) in dimethylformamide was added. The reaction mixture was stirred for another hour and quenched with a saturated aqueous ammonium chloride solution. The organic phase was dried, concentrated under reduced pressure, and purified by silica gel column chromatography with eluent C to give the title compound **1k** (130 mg, yield: 66.8%). MS m/z (ESI): 563.0[M+1].

### Step 10

### (±)-rel-4-((1S,3S,5R)-3-Ethoxy-8-((5-methoxy-7-methyl-1H-indol-4-yl)methyl)-8-azabicyclo[3.2.1]octan-1-yl)benzoic acid 1

Compound **1k** (130 mg, 231.03 µmol) was dissolved in 6 mL of a mixed solution of tetrahydrofuran, methanol, and water (V:V:V = 1:1:1). Lithium hydroxide monohydrate (58 mg, 1.38 mmol) was added. The reaction mixture was stirred at 70 °C for 3 h. The reaction mixture was concentrated, diluted with a small amount of methanol, and purified by preparative high performance liquid chromatography (Waters 2545, column: Sharpsil-T C18, 250 × 50 mm, 8 µm; mobile phase A: water (containing 10 mmol/L ammonium bicarbonate); mobile phase B: acetonitrile; 18 min gradient: 20%-38%, flow rate: 80 mL/min) to give the title compounds **1** (4 mg, yield: 3.86%) and **2** (5 mg, yield: 4.82%).

### Compound 1:

Preparative high performance liquid chromatography: retention time 17.28 min.

MS m/z (ESI): 449.1 [M+1]. ¹H NMR (500 MHz, CD₃OD): δ 8.16-8.14 (m, 2H), 7.69 (br, 2H), 7.35-7.34 (m, 1H), 6.84 (s, 1H), 6.34 (br, 1H), 4.20-4.03 (m, 3H), 3.93 (s, 3H), 3.71-3.58 (m, 1H), 3.51-3.34 (m, 2H), 3.32-2.96 (m, 2H), 2.73-2.68 (m,3H), 2.54 (s, 3H), 2.25-2.04 (m, 3H), 1.25-1.22 m, 3H).

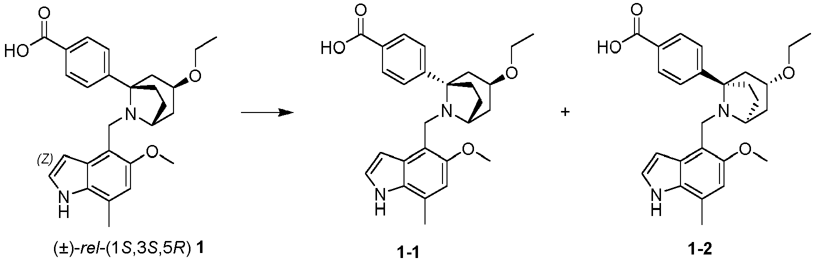

Compound **1** (100 mg, 222.93 µmol) was purified by preparative chiral chromatography (resolution conditions: preparative chiral column CHIRALPAK IG, 5 µm, 20 mm × 250 mm (Phenomenex); mobile phase 1: n-hexane (80%); mobile phase 2: containing 0.1% diethylamine, 0.1% trifluoroacetic acid, ethanol (20%), flow rate: 20 mL/min), and the corresponding fractions were collected and concentrated under reduced pressure to give the title compounds **1-1** (35 mg, yield: 35%) and **1-2** (33 mg, yield: 33%).

### Compound 1-1 (compound I, 4-((1S,3S,5R)-3-ethoxy-8-((5-methoxy-7-methyl-1H-indol-4-yl)methyl)-8-azabicyclo[3.2.1]octan-1-yl)benzoic acid):

MS m/z (ESI): 449.1 [M+1]. Chiral HPLC analysis: retention time 7.946 min, chiral purity: 100% (column: CHIRALPAK IG, 5 µm, 20 mm × 250 mm (Phenomenex); mobile phase 1: n-hexane (80%); mobile phase 2: containing 0.1% diethylamine, 0.1% trifluoroacetic acid, ethanol (20%), flow rate: 1 mL/min).

¹H NMR (500 MHz, MeOD) δ 8.16-8.15 (m, 2H), 7.69 (br, 2H), 7.34 (br, 1H), 6.83 (s, 1H), 6.33 (br, 1H), 4.22-4.12 (m, 2H), 4.03-4.00 (m, 1H), 3.93 (s, 3H), 3.71-3.51 (m, 1H), 3.50-3.35 (m, 2H), 3.32-2.96 (m, 2H), 2.73-2.53 (m, 3H), 2.51 (s, 3H), 2.21-2.05 (m, 3H), 1.35-1.22 (m, 3H).

### Example 2. Inhibitory Effect of Compound I on Enzyme Activity of Factor B

### I. Experimental materials and instruments

1. Recombinant human complement factor B protein (expressed by Nanjing GenScript Biotech Co., Ltd.)
2. Recombinant human complement factor D protein (1824-SE-010, R&D system)
3. Human complement factor C3 (204885-250UGCN, EMDmillipore)
4. Cobra venom factor (CVF) (A600, Quidel)
5. StartingBlock^{™} T20 (TBS) blocking buffer (37543, Thermo Fisher)
6. Goat anti-mouse IgG heavy chain + light chain (horseradish peroxidase-labeled) (ab205719, Abcam)
7. Anti-C3a/C3a des Arg antibody, clone number [2991] (ab11873, Abcam)
8. QuantaBlu^{™} fluorogenic peroxidase substrate kit (15169, Thermo Fisher)
9. Amphoteric surfactant (CHAPS) (C3023, Sigma)
10. Magnesium chloride solution (M1028-100ML, Sigma)
11. Sodium carbonate Na₂CO₃ (10019260, Hushi)
12. Sodium bicarbonate NaHCO₃ (10018960, Hushi)
13. Tween 20 (P7949-500ML, Sigma)
14. 20× PBS buffer (B548117-0500, Sangon)
15. 96-well low-volume white plate (66PL96025, Cisbio)
16. 96-well adsorption black plate (437111, Thermo Fisher)
17. Phosphate-buffered saline (B320, Shanghai BasalMedia Technologies Co., Ltd.)
18. Sterile purified water (made in-house by Shanghai Hengrui)
19. 96-well formulating plate (3795, Corning)
20. Constant-temperature incubator (Shanghai Yiheng Scientific Instruments Co., Ltd.)
21. Flexstation3 microplate reader (Molecular Device)

### II. Experimental procedure

The functioning of the human complement factor B protein as a protease requires binding to human complement factor C3 to form a complex. Human complement factor B is hydrolyzed into the Ba and Bb fragments by the human complement factor D protein. Bb and the C3b fragment of human complement factor C3 form a complex C3bBb, i.e., C3 convertase. Only the formation of the complex can enable human complement factor B to function as a protease. C3bBb continues to hydrolyze C3 into the C3a and C3b fragments. C3b and C3bBb form a complex C3bBbC3b, i.e., C5 convertase, and the C3a fragment is released. Assays for the C3a des Arg epitope generated after C3 is cleaved can be used to evaluate the efficiency of C3 being hydrolyzed, i.e., the C3bBb enzyme activity, and thereby to evaluate the effects of the compounds on the C3bBb enzyme. Since C3b is unstable *in vitro,* cobra venom factor (hereinafter referred to as CVF) was used in place of C3b to form a complex with human complement factor B. It functions in the same way as C3b.

The coding gene for the AA128-2422 amino acid segment of the human complement factor B protein (NM_001710.6) was subjected to codon optimization, gene synthesis, and cloning into the pcDNA3.4 vector by Nanjing GenScript Biotech Co., Ltd. and was expressed in HD CHO-S cells, and the product was purified. The recombinant human complement factor B protein obtained from the purification was aliquoted and then stored in a -80 °C freezer.

Cleavage reactions of the human complement factor B protein: The recombinant human complement factor D protein was diluted 10-fold with PBS (pH 7.4) and stored on ice for later use. The recombinant human complement factor D protein, the recombinant human complement factor B protein, and CVF were added to a reaction buffer (PBS pH 7.4, 10 mM MgCl₂, 0.05% CHAPS) to final concentrations of 300 nM, 1 µM, and 1 µM, respectively. After they were well mixed, the mixture was reacted in a constant-temperature incubator at 37 °C for 3 h to give a complex of CVF and the post-cleavage fragment Bb of the recombinant human complement factor B protein (hereinafter referred to as CVF:Bb).

A 100 mM Na₂CO₃ solution and a 100 mM NaHCO₃ solution were prepared and mixed in a ratio of Na₂CO₃ to NaHCO₃ of 3:7 (v/v) to adjust the pH to 9.5. The mixture was stored at room temperature for later use.

20 mM test compounds, dissolved in 100% DMSO, were serially diluted with 100% DMSO to 2000, 500, 125, 31.25, 7.8125, 0.488281, 0.12207, 0.030518, and 0.007629 µM, with the blank well containing 100% DMSO. The compounds and 100% DMSO were then 20-fold diluted in C3 reaction buffer (PBS pH 7.4, 1 mM MgCl₂, 0.05% CHAPS).

Cleavage reactions of the C3 protein: In a 96-well low-volume white plate, 10-µL reaction systems were prepared by adding CVF:Bb (to a final concentration of 2 nM) and 1 µL of the above test compounds and DMSO diluted in C3 reaction buffer to C3 reaction buffer (PBS pH 7.4, 1 mM MgCl₂, 0.05% CHAPS). The plate was incubated at room temperature for 1 h. The final concentrations of the test compounds were 10,000, 2500, 625, 156.25, 39.0625, 9.765625, 2.441406, 0.6103515, and 0.152588 nM, respectively. Human complement factor C3 was added to the reaction systems to a final concentration of 500 nM. After they were well mixed, the mixtures were reacted in a constant-temperature incubator at 37 °C for 2 h. Among the reaction mixtures, the reaction well that contained only 500 nM human complement factor C3 was used as a negative control. To a 96-well adsorption black plate, 97 µL of the carbonic acid buffer (pH 9.5) was added, and the C3 protein cleavage reaction mixtures were added at 3 µL/well. After they were well mixed, the plate was sealed and incubated at 4 °C overnight.

C3a des Arg assays: The plate was washed 3 times with 300 µL/well TBST (0.05% tween 20) solution, the StartingBlock^{™} T20 (TBS) blocking buffer was added at 300 µL/well, and the plate was incubated at 37 °C for 5 min. The plate was washed 3 times with 300 µL/well PBST solution, the anti-C3a/C3a des Arg antibody [2991] was 1:1000 diluted in PBST solution and added at 100 µL/well, and the plate was incubated at 37 °C for 1 h. The plate was washed 3 times with 300 µL/well PBST solution, the goat anti-mouse IgG H&L (HRP) antibody was 1:5000 diluted in PBST solution and added at 100 µL/well, and the plate was incubated at 37 °C for 30 min. A QuantaBlu^{™} fluorogenic peroxidase substrate kit substrate was prepared by diluting 1 part of the QuantaBlu^{™} stable peroxide solution with 9 parts of the QuantaBlu^{™} substrate solution. The plate was washed 3 times with 300 µL/well PBST solution, and dried after the last wash. The substrate was added at 100 µL/well, and the plate was incubated at room temperature for 20 min. After the QuantaBlu^{™} stop solution was added at 100 µL/well, the fluorescence readings were taken on Flexstation, with the excitation light wavelength Ex set to 320 nM and the emission light wavelength Em set to 460 nM, cutoff 455.

The inhibition rate was calculated using the following formula: Inhibition rate = {1- (RFUtest compound - RFUnegative control well) / (RFUblank well - RFUnegative control well)} × 100%

Inhibition curves were plotted using Graphpad Prism software according to the concentrations of the compounds and the corresponding inhibition rates, and the concentrations of the compounds at which the inhibition rate reached 50%, i.e., IC₅₀ values, were calculated.

Conclusion: The IC₅₀ for the inhibitory activity of compound I against the Factor B enzyme was 1.3 nM, indicating that compound I has a very good inhibitory effect on the Factor B enzyme.

### Example 3. Preparation of Crystal Form I of Maleate

About 8 mg of compound I was weighed out and dissolved in 0.2 mL of acetonitrile, and a maleic acid solution (2 mol/L, 9.8 µL) was added. The compound was slurried overnight at room temperature, and 0.6 mL of isopropyl ether was added. The mixture was stirred for crystallization and centrifuged, and the resulting solid was dried *in vacuo* to give a product.

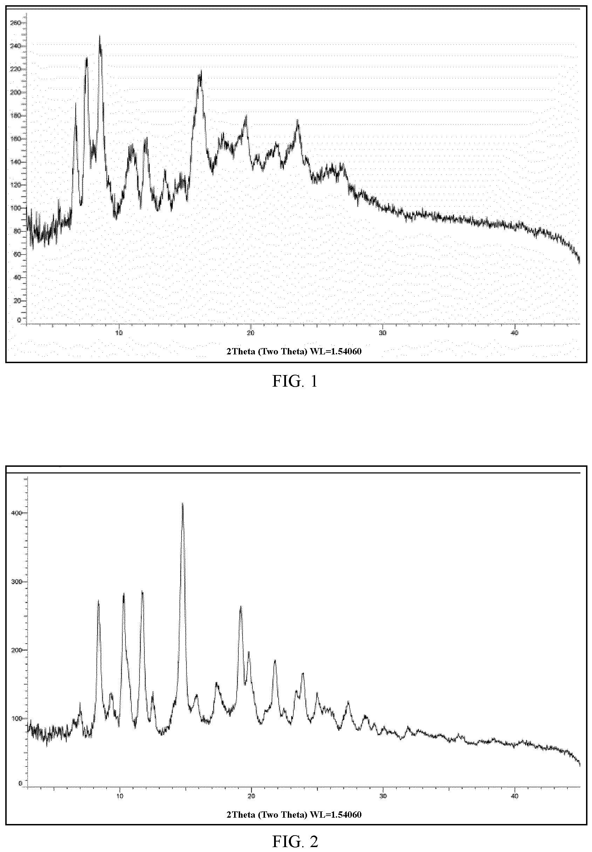

According to an X-ray powder diffraction analysis, the product was defined as the crystal form I of the maleate. An XRPD pattern of the crystal form is shown in FIG. 1, and its characteristic peak positions are shown in Table 1. A DSC profile shows an endothermic peak at 156.79 °C. A TGA profile shows a weight loss of 3.22% from 30 °C to 145 °C.

**Table 1. Peak positions of the crystal form I of the maleate**

| Peak No. | 2*θ* value [° or degrees] | D [Å] | Relative intensity% |
|---|---|---|---|
| 1 | 6.716 | 13.15131 | 70.2 |
| 2 | 7.577 | 11.65841 | 92.6 |
| 3 | 8.096 | 10.91244 | 35.8 |
| 4 | 8.604 | 10.26848 | 100.0 |
| 5 | 9.255 | 9.54754 | 15.8 |
| 6 | 11.012 | 8.02779 | 36.1 |
| 7 | 12.059 | 7.33337 | 28.8 |
| 8 | 13.508 | 6.54999 | 16.3 |
| 9 | 14.738 | 6.00599 | 11.9 |
| 10 | 16.155 | 5.48201 | 58.7 |
| 11 | 17.900 | 4.95128 | 17.2 |
| 12 | 19.657 | 4.51249 | 25.7 |
| 13 | 21.450 | 4.13932 | 2.0 |
| 14 | 21.977 | 4.04121 | 7.9 |
| 15 | 22.891 | 3.88192 | 3.2 |
| 16 | 23.523 | 3.77895 | 26.6 |
| 17 | 24.226 | 3.67088 | 5.1 |
| 18 | 26.159 | 3.40386 | 5.2 |
| 19 | 27.002 | 3.29943 | 12.4 |

### Example 4. Preparation of Crystal Form I of Phosphate

About 8 mg of compound I was weighed out. 0.2 mL of acetonitrile was added, and a phosphoric acid solution (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form I of the phosphate. An XRPD pattern of the crystal form is shown in FIG. 2, and its characteristic peak positions are shown in Table 2. A DSC profile shows an exothermic peak at 195.83 °C. A TGA profile shows a weight loss of 2.80% from 30 °C to 150 °C.

**Table 2. Peak positions of the crystal form I of the phosphate**

| Peak No. | 2*θ* value [° or degrees] | D [Å] | Relative intensity% |
|---|---|---|---|
| 1 | 6.985 | 12.64549 | 12.9 |
| 2 | 8.405 | 10.51147 | 57.0 |
| 3 | 9.317 | 9.48409 | 13.6 |
| 4 | 10.292 | 8.58812 | 56.5 |
| 5 | 11.735 | 7.53529 | 60.3 |
| 6 | 12.518 | 7.06538 | 15.6 |
| 7 | 14.780 | 5.98892 | 100.0 |
| 8 | 15.802 | 5.60358 | 10.6 |
| 9 | 17.375 | 5.09992 | 14.9 |
| 10 | 19.185 | 4.62262 | 49.4 |
| 11 | 19.787 | 4.48317 | 29.1 |
| 12 | 21.782 | 4.07691 | 26.2 |
| 13 | 23.401 | 3.79844 | 11.6 |
| 14 | 23.887 | 3.72215 | 19.9 |
| 15 | 24.964 | 3.56401 | 11.4 |
| 16 | 26.018 | 3.42193 | 4.4 |
| 17 | 27.319 | 3.26194 | 9.4 |
| 18 | 28.619 | 3.11663 | 3.0 |
| 19 | 29.357 | 3.03994 | 1.5 |
| 20 | 30.024 | 2.97383 | 0.3 |
| 21 | 31.852 | 2.80727 | 1.6 |

### Example 5. Preparation of Crystal Form I of Phosphate

About 8 mg of compound I was weighed out. 0.2 mL of acetone was added, and a phosphoric acid solution (2 mol/L, 9.8 µL) was added. The mixture was stirred for crystallization and centrifuged, and the resulting solid was dried *in vacuo* to give a product.

The product was the crystal form I of the phosphate, as identified by X-ray powder diffraction.

### Example 6. Preparation of Crystal Form II of Phosphate

About 8 mg of compound I was weighed out. 0.2 mL of ethyl acetate was added, and a solution of phosphoric acid in ethanol (2 mol/L, 9.8 µL) was added. The mixture was stirred for crystallization and centrifuged, and the resulting solid was dried *in vacuo* to give a product.

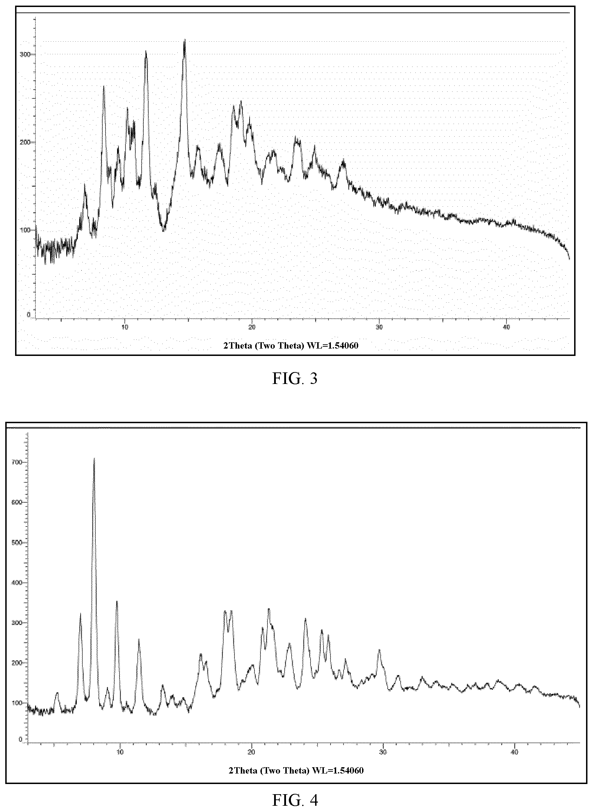

According to an X-ray powder diffraction analysis, the product was defined as the crystal form II of the phosphate. An XRPD pattern of the crystal form is shown in FIG. 3, and its characteristic peak positions are shown in Table 3. The phosphate ion content was 17.33%, as determined by ion chromatography. A DSC profile shows an endothermic peak at 146.30 °C. A TGA profile shows a weight loss of 1.09% from 30 °C to 175 °C.

**Table 3. Peak positions of the crystal form II of the phosphate**

| Peak No. | 2*θ* value [° or degrees] | D [Å] | Relative intensity (%) |
|---|---|---|---|
| 1 | 6.859 | 12.87704 | 31.0 |
| 2 | 8.351 | 10.57931 | 83.4 |
| 3 | 8.834 | 10.00236 | 32.5 |
| 4 | 9.479 | 9.32259 | 41.2 |
| 5 | 10.188 | 8.67558 | 64.9 |
| 6 | 10.682 | 8.27550 | 56.9 |
| 7 | 11.660 | 7.58331 | 100.0 |
| 8 | 12.383 | 7.14218 | 15.6 |
| 9 | 14.722 | 6.01215 | 96.3 |
| 10 | 15.741 | 5.62531 | 25.2 |
| 11 | 17.479 | 5.06978 | 20.6 |
| 12 | 18.549 | 4.77959 | 41.3 |
| 13 | 19.122 | 4.63759 | 43.3 |
| 14 | 19.763 | 4.48865 | 28.3 |
| 15 | 21.309 | 4.16630 | 9.7 |
| 16 | 21.731 | 4.08640 | 12.3 |
| 17 | 22.434 | 3.95993 | 1.5 |
| 18 | 23.418 | 3.79572 | 17.9 |
| 19 | 23.804 | 3.73495 | 17.5 |
| 20 | 24.929 | 3.56895 | 15.9 |
| 21 | 27.248 | 3.27020 | 10.8 |

### Example 7. Preparation of Crystal Form II of Phosphate

About 40 mg of compound I was weighed out. 0.75 mL of acetone was added, and a phosphoric acid solution (2 mol/L, 47 µL) was added. The mixture was stirred for crystallization and centrifuged, and the resulting solid was dried *in vacuo* to give a product.

The product was the crystal form II of the phosphate, as identified by X-ray powder diffraction.

### Example 8. Preparation of Crystal Form III of Phosphate

About 8 mg of compound I was weighed out. 0.2 mL of isopropanol was added, and a solution of phosphoric acid in ethanol (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form III of the phosphate. An XRPD pattern of the crystal form is shown in FIG. 4, and its characteristic peak positions are shown in Table 4. The phosphate ion content was 16.72%, as determined by ion chromatography. A DSC profile shows endothermic peaks at 66.14 °C and 143.48 °C and an exothermic peak at 182.62 °C. A TGA profile shows a weight loss of 6.16% from 30 °C to 150 °C. DVS testing shows that the sample had a hygroscopic weight gain of about 4.77% under normal storage conditions (i.e., 25 °C and 60% RH), a hygroscopic weight gain of about 5.44% under accelerated experimental conditions (i.e., 70% RH), and a hygroscopic weight gain of about 7.20% under extreme conditions (i.e., 90% RH). Moreover, according to a crystal form re-identification, the crystal form did not change after DVS testing.

**Table 4. Peak positions of the crystal form III of the phosphate**

| Peak No. | 2*θ* value [° or degrees] | D [Å] | Relative intensity (%) |
|---|---|---|---|
| 1 | 5.207 | 16.95749 | 7.0 |
| 2 | 7.003 | 12.61306 | 38.7 |
| 3 | 8.028 | 11.00398 | 100.0 |
| 4 | 9.052 | 9.76191 | 7.3 |
| 5 | 9.778 | 9.03845 | 43.3 |
| 6 | 10.520 | 8.40208 | 3.4 |
| 7 | 11.453 | 7.71995 | 28.1 |
| 8 | 13.248 | 6.67755 | 9.8 |
| 9 | 13.965 | 6.33627 | 5.6 |
| 10 | 14.817 | 5.97385 | 3.8 |
| 11 | 16.136 | 5.48856 | 18.7 |
| 12 | 16.528 | 5.35903 | 14.2 |
| 13 | 18.028 | 4.91643 | 31.4 |
| 14 | 18.455 | 4.80374 | 31.6 |
| 15 | 20.047 | 4.42574 | 7.0 |
| 16 | 20.848 | 4.25733 | 21.4 |
| 17 | 21.346 | 4.15918 | 29.1 |
| 18 | 21.582 | 4.11425 | 20.9 |
| 19 | 22.900 | 3.88042 | 16.2 |
| 20 | 24.107 | 3.68872 | 25.9 |
| 21 | 25.335 | 3.51261 | 19.3 |
| 22 | 25.846 | 3.44429 | 18.0 |
| 23 | 26.686 | 3.33781 | 3.9 |
| 24 | 27.140 | 3.28298 | 9.1 |
| 25 | 28.443 | 3.13548 | 0.8 |
| 26 | 29.722 | 3.00344 | 14.4 |
| 27 | 31.254 | 2.85955 | 3.3 |
| 28 | 33.047 | 2.70844 | 2.6 |
| 29 | 34.136 | 2.62446 | 1.0 |
| 30 | 35.366 | 2.53595 | 0.4 |
| 31 | 36.491 | 2.46034 | 0.4 |
| 32 | 37.896 | 2.37224 | 0.6 |
| 33 | 38.740 | 2.32252 | 1.6 |
| 34 | 40.391 | 2.23127 | 0.9 |
| 35 | 41.446 | 2.17692 | 1.0 |

### Example 9. Preparation of Crystal Form IV of Phosphate

About 30 mg of compound I was weighed out and dissolved in 0.5 mL of acetonitrile. The solution was heated to 40 °C, and 8.2 mg of 85% phosphoric acid was added. The mixture was cooled to room temperature, stirred for 16 h, and filtered, and the filter cake was collected and dried *in vacuo* at 60 °C for 4 h to give a product.

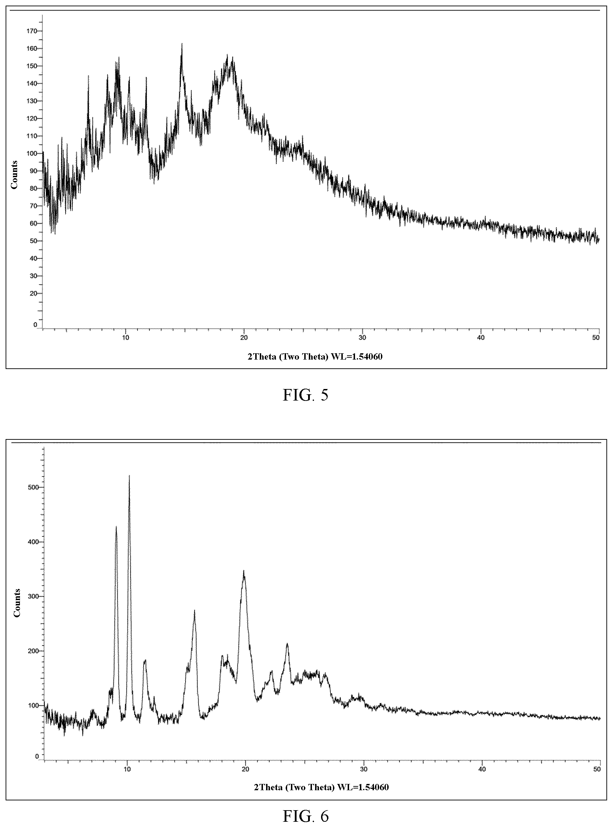

According to an X-ray powder diffraction analysis, the product was defined as the crystal form IV of the phosphate. An XRPD pattern of the crystal form is shown in FIG. 5, and its characteristic peak positions are shown in Table 5.

**Table 5. Peak positions of the crystal form IV of the phosphate**

| Peak No. | 2θ value [° or degrees] | D [Å] | Relative intensity (%) |
|---|---|---|---|
| 1 | 6.851 | 12.89254 | 79.50 |
| 2 | 8.446 | 10.46006 | 63.00 |
| 3 | 10.283 | 8.59566 | 65.30 |
| 4 | 11.706 | 7.55343 | 64.50 |
| 5 | 14.757 | 5.99805 | 100.00 |

### Example 10. Preparation of Crystal Form V of Phosphate

30 mg of compound I was weighed out and dissolved in 1 mL of ethanol. The solution was heated to 40 °C, and 85% phosphoric acid (8.2 mg, 66.88 µmol) was added. The mixture was cooled to room temperature, stirred for 16 h, and filtered, and the filter cake was collected and dried *in vacuo* at 60 °C for 4 h to give a product.

According to an X-ray powder diffraction analysis, the product was defined as the crystal form V of the phosphate. An XRPD pattern of the crystal form is shown in FIG. 6, and its characteristic peak positions are shown in Table 6. A DSC profile shows endothermic peaks at 48.64 °C and 223.41 °C and an exothermic peak at 194.80 °C. A TGA profile shows a weight loss of 2.47% from 30 °C to 100 °C and a weight loss of 2.76% from 100 °C to 250 °C.

**Table 6. Peak positions of the crystal form V of the phosphate**

| Peak No. | 2θ value [° or degrees] | D [Å] | Relative intensity |
|---|---|---|---|
| 1 | 8.644 | 10.22083 | 11.30 |
| 2 | 9.082 | 9.72906 | 80.00 |
| 3 | 10.179 | 8.68351 | 100.00 |
| 4 | 11.505 | 7.68507 | 23.80 |
| 5 | 15.168 | 5.83667 | 21.50 |
| 6 | 15.692 | 5.64259 | 43.40 |
| 7 | 18.038 | 4.91376 | 19.80 |
| 8 | 19.849 | 4.46933 | 52.40 |
| 9 | 22.176 | 4.00535 | 7.70 |
| 10 | 23.525 | 3.77869 | 18.10 |

### Example 11. Preparation of Crystal Form I of p-Toluenesulfonate

About 8 mg of compound I was weighed out and dissolved in 0.2 mL of ethanol, and a p-toluenesulfonic acid solution (2 mol/L, 9.8 µL) was added. The mixture was stirred overnight, and 0.8 mL of isopropyl ether was added. The mixture was stirred for crystallization and centrifuged, and the resulting solid was dried *in vacuo* to give a product.

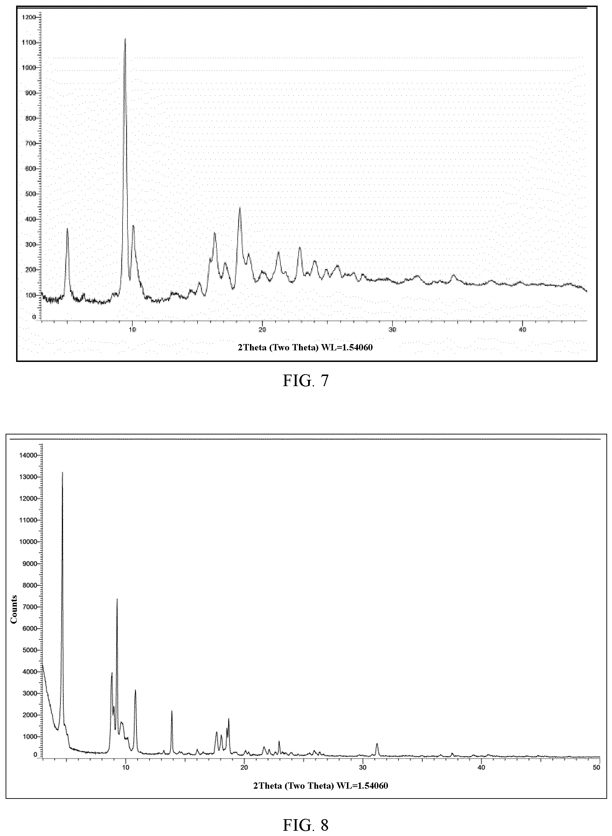

According to an X-ray powder diffraction analysis, the product was defined as the crystal form I of the p-toluenesulfonate. An XRPD pattern of the crystal form is shown in FIG. 7, and its characteristic peak positions are shown in Table 7. The p-toluenesulfonate ion content was 30.75%, as determined by ion chromatography. A DSC profile shows endothermic peaks at 57.15 °C and 180.78 °C. A TGA profile shows a weight loss of 2.07% from 30 °C to 145 °C.

DVS testing shows that the sample had a hygroscopic weight gain of about 1.22% under normal storage conditions (i.e., 25 °C and 60% RH), a hygroscopic weight gain of about 1.37% under accelerated experimental conditions (i.e., 70% RH), and a hygroscopic weight gain of about 1.87% under extreme conditions (i.e., 90% RH). Moreover, according to a crystal form re-identification, the crystal form did not change after DVS testing.

**Table 7. Peak positions of the crystal form I of the p-toluenesulfonate**

| Peak No. | 2*θ* value [° or degrees] | D [Å] | Relative intensity (%) |
|---|---|---|---|
| 1 | 5.001 | 17.65696 | 27.7 |
| 2 | 9.445 | 9.35600 | 100.0 |
| 3 | 10.071 | 8.77568 | 27.8 |
| 4 | 15.165 | 5.83774 | 4.1 |
| 5 | 15.984 | 5.54039 | 12.7 |
| 6 | 16.331 | 5.42334 | 21.6 |
| 7 | 17.123 | 5.17419 | 9.5 |
| 8 | 18.251 | 4.85694 | 29.1 |
| 9 | 18.941 | 4.68150 | 10.7 |
| 10 | 19.939 | 4.44949 | 3.3 |
| 11 | 21.229 | 4.18195 | 10.2 |
| 12 | 22.871 | 3.88513 | 11.7 |
| 13 | 24.002 | 3.70460 | 6.1 |
| 14 | 24.859 | 3.57888 | 2.5 |
| 15 | 25.755 | 3.45633 | 3.7 |
| 16 | 27.073 | 3.29102 | 1.8 |
| 17 | 27.740 | 3.21330 | 1.1 |
| 18 | 31.992 | 2.79525 | 1.6 |
| 19 | 34.734 | 2.58067 | 2.2 |

### Example 12. Preparation of Crystal Form I of p-Toluenesulfonate

Compound I was weighed out, and a solvent and a 2 mol/L p-toluenesulfonic acid solution were added. Crystallization was performed to give a product. The crystal form was determined by X-ray powder diffraction, as shown in Table 8.

**Table 8. Preparation of the crystal form I of the p-toluenesulfonate**

| No. | Feed amount and solvent | Crystal form |
|---|---|---|
| 1 | About 8 mg of compound I was weighed out and dissolved in 0.2 mL of isopropanol, and a p-toluenesulfonic acid solution (2 mol/L, 9.8 µL) was added. The mixture was stirred overnight, and 0.8 mL of isopropyl ether was added. The mixture was stirred for crystallization. | Crystal form I of p-toluenesulfonate |
| 2 | About 8 mg of compound I was weighed out and dissolved in 0.2 mL of ethyl acetate, and a p-toluenesulfonic acid solution (2 mol/L, 9.8 µL) was added. The mixture was stirred overnight, and 0.8 mL of isopropyl ether was added. The mixture was stirred for crystallization. | Crystal form I of p-toluenesulfonate |
| 3 | About 100 mg of compound I was weighed out. 0.8 mL of acetonitrile was added, and a solution of p-toluenesulfonic acid in tetrahydrofuran (2 mol/L, 140 µL) was added. The mixture was stirred for crystallization. | Crystal form I of p-toluenesulfonate |

### Example 13. Preparation of Crystal Form II of p-Toluenesulfonate

About 938 mg of compound I was weighed out and dissolved in 40 mL of isopropanol. The mixture was stirred at 40 °C until the compound was completely dissolved. The solution was heated to 60 °C, and 398 mg of p-toluenesulfonic acid monohydrate was added. The mixture was stirred for 1 h, then cooled to room temperature, and filtered, and the filter cake was collected and dried *in vacuo* at 60 °C for 4 h to give a product.

According to an X-ray powder diffraction analysis, the product was defined as the crystal form II of the p-toluenesulfonate. An XRPD pattern of the crystal form is shown in FIG. 8, and its characteristic peak positions are shown in Table 9. A DSC profile shows endothermic peaks at 104.06 °C and 181.38 °C and an exothermic peak at 188.49 °C. A TGA profile shows a weight loss of 3.36% from 40 °C to 160 °C and a weight loss of 3.84% from 160 °C to 270 °C.

**Table 9. Peak positions of the crystal form II of the p-toluenesulfonate**

| Peak No. | 2*θ* value [° or degrees] | D [Å] | Relative intensity (%) |
|---|---|---|---|
| 1 | 4.661 | 18.94459 | 100.00 |
| 2 | 8.837 | 9.99804 | 30.40 |
| 3 | 9.269 | 9.53382 | 58.20 |
| 4 | 9.663 | 9.14573 | 11.70 |
| 5 | 10.099 | 8.75216 | 5.70 |
| 6 | 10.818 | 8.17172 | 24.40 |
| 7 | 13.216 | 6.69403 | 1.40 |
| 8 | 13.889 | 6.37098 | 16.20 |
| 9 | 16.026 | 5.52584 | 2.00 |
| 10 | 17.674 | 5.01430 | 8.40 |
| 11 | 18.065 | 4.90652 | 7.20 |
| 12 | 18.541 | 4.78165 | 9.80 |
| 13 | 18.686 | 4.74497 | 13.50 |
| 14 | 20.107 | 4.41251 | 1.70 |
| 15 | 21.669 | 4.09797 | 3.10 |
| 16 | 22.106 | 4.01783 | 2.00 |
| 17 | 22.940 | 3.87363 | 5.20 |
| 18 | 25.894 | 3.43807 | 1.60 |
| 19 | 31.193 | 2.86507 | 4.90 |

### Example 14. Preparation of Crystal Form III of p-Toluenesulfonate

15 mg of the crystal form II of the p-toluenesulfonate compound was dispersed in 1 mL of methyl tert-butyl ether. The dispersion was stirred for 72 h and filtered, and the filter cake was collected and dried *in vacuo* at 60 °C for 4 h to give a product.

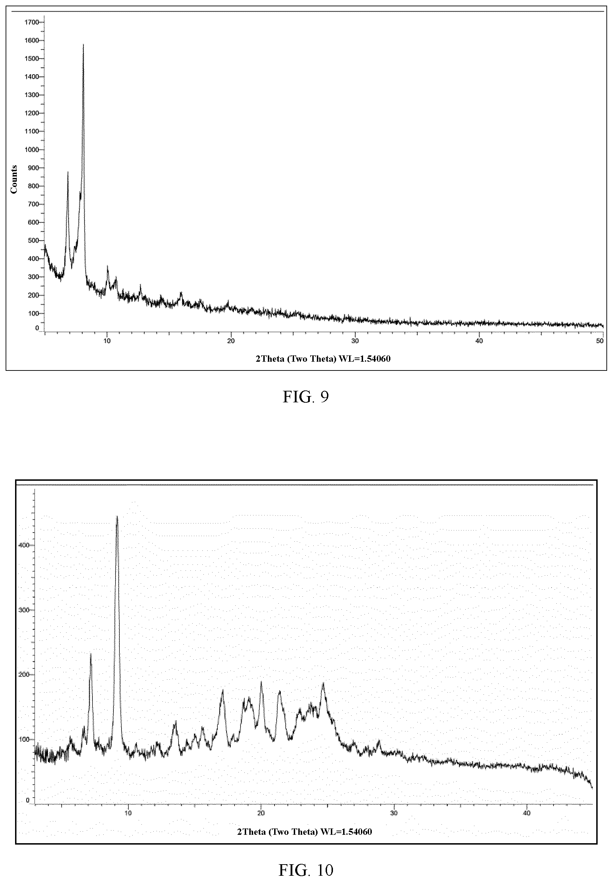

According to an X-ray powder diffraction analysis, the product was defined as the crystal form III of the p-toluenesulfonate. An XRPD pattern of the crystal form is shown in FIG. 9, and its characteristic peak positions are shown in Table 10.

**Table 10. Peak positions of the crystal form III of the p-toluenesulfonate**

| Peak No. | 2*θ* value [° or degrees] | D[Å] | Relative intensity (%) |
|---|---|---|---|
| 1 | 6.847 | 12.90015 | 45.10 |
| 2 | 7.366 | 11.99200 | 14.00 |
| 3 | 8.093 | 10.91591 | 100.00 |
| 4 | 10.064 | 8.78217 | 9.40 |
| 5 | 10.687 | 8.27138 | 4.40 |
| 6 | 12.705 | 6.96167 | 5.00 |
| 7 | 15.973 | 5.54412 | 3.90 |

### Example 15. Preparation of Crystal Form I of Sulfate

About 8 mg of compound I was weighed out. 0.2 mL of acetonitrile was added, and a sulfuric acid solution (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form I of the sulfate. An XRPD pattern of the crystal form is shown in FIG. 10, and its characteristic peak positions are shown in Table 11. A DSC profile shows endothermic peaks at 52.82 °C and 105.48 °C and an exothermic peak at 190.22 °C. A TGA profile shows a weight loss of 6.02% from 30 °C to 195 °C.

**Table 11. Peak positions of the crystal form I of the sulfate**

| Peak No. | 2*θ* value [° or degrees] | D[Å] | Relative intensity (%) |
|---|---|---|---|
| 1 | 6.696 | 13.19028 | 9.7 |
| 2 | 7.192 | 12.28059 | 39.6 |
| 3 | 9.176 | 9.62981 | 100.0 |
| 4 | 10.696 | 8.26445 | 1.1 |
| 5 | 12.172 | 7.26543 | 3.5 |
| 6 | 13.515 | 6.54627 | 9.5 |
| 7 | 14.421 | 6.13699 | 1.4 |
| 8 | 15.019 | 5.89418 | 5.0 |
| 9 | 15.576 | 5.68464 | 6.8 |
| 10 | 17.116 | 5.17638 | 21.2 |
| 11 | 18.728 | 4.73420 | 15.4 |
| 12 | 19.060 | 4.65256 | 14.5 |
| 13 | 20.025 | 4.43055 | 22.2 |
| 14 | 21.398 | 4.14916 | 18.6 |
| 15 | 22.893 | 3.88156 | 9.2 |
| 16 | 23.804 | 3.73495 | 10.9 |
| 17 | 24.676 | 3.60492 | 21.7 |
| 18 | 27.002 | 3.29943 | 2.0 |
| 19 | 28.830 | 3.09431 | 3.1 |

### Example 16. Preparation of Crystal Form I of Sulfate

About 8 mg of compound I was weighed out. 0.2 mL of ethanol was added, and a sulfuric acid solution (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form I of the sulfate.

### Example 17. Preparation of Crystal Form II of Sulfate

About 8 mg of compound I was weighed out. 0.2 mL of acetone was added, and a sulfuric acid solution (2 mol/L, 9.8 µL) was added. The mixture was stirred for crystallization and centrifuged, and the resulting solid was dried *in vacuo* to give a product.

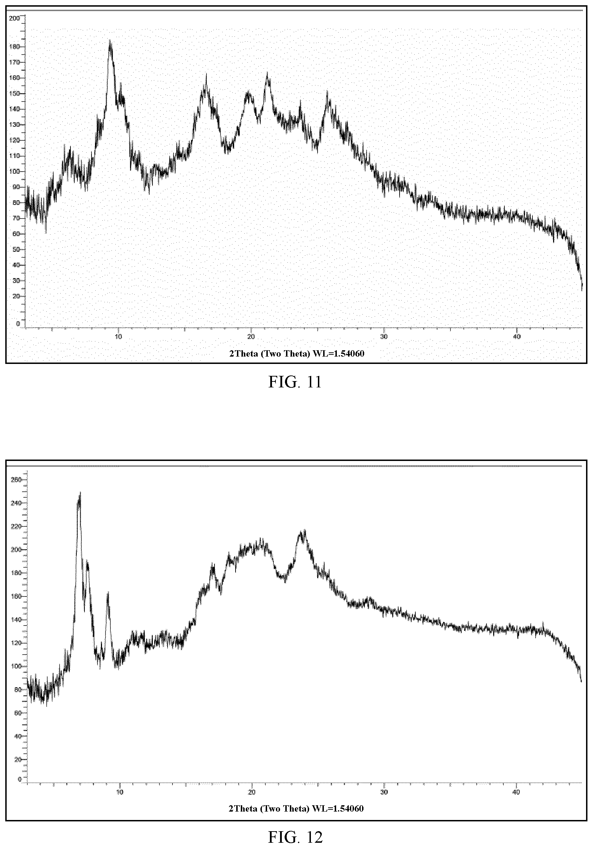

According to an X-ray powder diffraction analysis, the product was defined as the crystal form II of the sulfate. An XRPD pattern of the crystal form is shown in FIG. 11, and its characteristic peak positions are shown in Table 12. A DSC profile shows an endothermic peak at 64.63 °C and an exothermic peak at 207.53 °C. A TGA profile shows a weight loss of 6.12% from 30 °C to 180 °C.

**Table 12. Peak positions of the crystal form II of the sulfate**

| Peak No. | 2*θ* value [° or degrees] | D[Å] | Relative intensity (%) |
|---|---|---|---|
| 1 | 6.268 | 14.08908 | 22.0 |
| 2 | 8.482 | 10.41596 | 42.4 |
| 3 | 9.473 | 9.32825 | 100.0 |
| 4 | 10.204 | 8.66179 | 63.2 |
| 5 | 16.565 | 5.34731 | 41.5 |
| 6 | 19.763 | 4.48865 | 22.9 |
| 7 | 21.204 | 4.18678 | 33.9 |
| 8 | 23.734 | 3.74585 | 7.9 |
| 9 | 25.702 | 3.46332 | 33.6 |

### Example 18. Preparation of Crystal Form III of Sulfate

About 40 mg of compound I was weighed out and dissolved in 0.75 mL of ethanol. A sulfuric acid solution (2 mol/L, 47 µL) was added, and 1 mL of isopropyl ether was added. The mixture was stirred for crystallization and centrifuged, and the resulting solid was dried *in vacuo* to give a product. According to an X-ray powder diffraction analysis, the product was defined as the crystal form III of the sulfate. An XRPD pattern of the crystal form is shown in FIG. 12, and its characteristic peak positions are shown in Table 13. The sulfate ion content was 15.31%, as determined by ion chromatography. A DSC profile shows endothermic peaks at 76.24 °C and 150.06 °C. A TGA profile shows a weight loss of 4.31% from 30 °C to 140 °C.

**Table 13. Peak positions of the crystal form III of the sulfate**

| Peak No. | 2*θ* value [° or degrees] | D[Å] | Relative intensity (%) |
|---|---|---|---|
| 1 | 6.946 | 12.71565 | 100.0 |
| 2 | 7.566 | 11.67522 | 56.0 |
| 3 | 9.098 | 9.71181 | 30.3 |
| 4 | 10.942 | 8.07920 | 9.8 |
| 5 | 11.786 | 7.50285 | 1.0 |
| 6 | 17.022 | 5.20480 | 12.4 |
| 7 | 18.182 | 4.87535 | 13.5 |
| 8 | 20.747 | 4.27793 | 11.0 |
| 9 | 23.699 | 3.75132 | 20.5 |
| 10 | 24.015 | 3.70263 | 18.2 |

### Example 19. Preparation of Crystal Form IV of Sulfate

About 8 mg of compound I was weighed out. 0.2 mL of isopropanol was added, and a solution of sulfuric acid in ethanol (2 mol/L, 9.8 µL) was added. The mixture was stirred for crystallization and centrifuged, and the resulting solid was dried *in vacuo* to give a product.

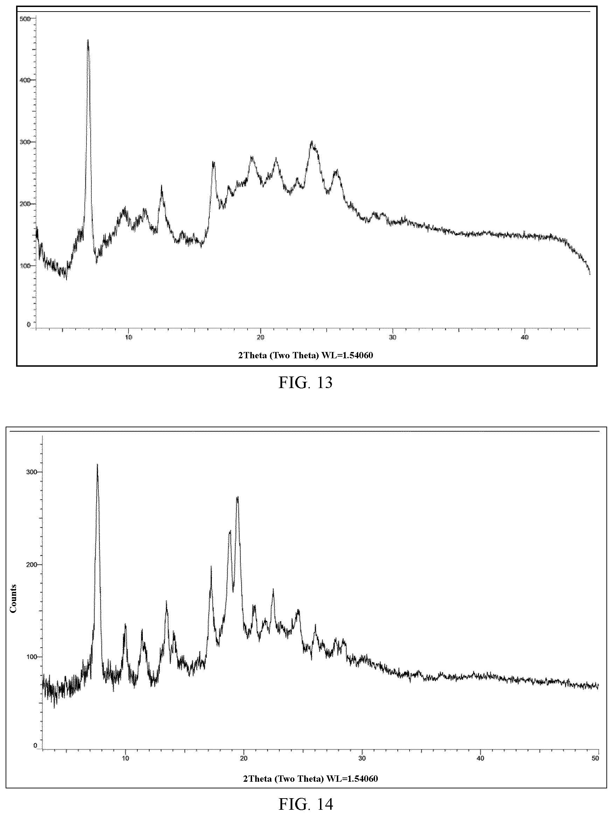

According to an X-ray powder diffraction analysis, the product was defined as the crystal form IV of the sulfate. An XRPD pattern of the crystal form is shown in FIG. 13, and its characteristic peak positions are shown in Table 14. The sulfate ion content was 15.72%, as determined by ion chromatography. A DSC profile shows endothermic peaks at 68.75 °C and 161.89 °C. A TGA profile shows a weight loss of 3.74% from 30 °C to 135 °C.

**Table 14. Peak positions of the crystal form IV of the sulfate**

| Peak No. | 2*θ* value [° or degrees] | D[Å] | Relative intensity (%) |
|---|---|---|---|
| 1 | 6.939 | 12.72904 | 100.0 |
| 2 | 9.672 | 9.13715 | 10.7 |
| 3 | 11.294 | 7.82857 | 10.5 |
| 4 | 12.539 | 7.05349 | 16.4 |
| 5 | 16.473 | 5.37698 | 28.4 |
| 6 | 17.584 | 5.03962 | 9.9 |
| 7 | 19.408 | 4.56983 | 17.3 |
| 8 | 21.169 | 4.19365 | 13.5 |
| 9 | 22.750 | 3.90559 | 3.0 |
| 10 | 23.962 | 3.71067 | 17.8 |
| 11 | 25.802 | 3.45016 | 11.7 |
| 12 | 28.689 | 3.10915 | 0.9 |

### Example 20. Preparation of Crystal Form IV of Sulfate

About 40 mg of compound I was weighed out. 0.75 mL of acetone was added, and a sulfuric acid solution (2 mol/L, 47 µL) was added. The mixture was stirred for crystallization and centrifuged, and the resulting solid was dried *in vacuo* to give a product.

The product was the crystal form IV of the sulfate, as identified by X-ray powder diffraction.

### Example 21. Preparation of Crystal Form V of Sulfate

About 8 mg of compound I was weighed out. 0.2 mL of ethyl acetate was added, and a solution of sulfuric acid in ethanol (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form V of the sulfate. An XRPD pattern of the crystal form is shown in FIG. 14, and its characteristic peak positions are shown in Table 15. The sulfate ion content was 11.65%, as determined by ion chromatography. A DSC profile shows endothermic peaks at 61.82 °C and 153.47 °C. A TGA profile shows a weight loss of 4.19% from 30 °C to 90 °C and a weight loss of 6.18% from 90 °C to 195 °C.

**Table 15. Peak positions of the crystal form V of the sulfate**

| Peak No. | 2θ value [° or degrees] | D[Å] | Relative intensity% |
|---|---|---|---|
| 1 | 7.624 | 11.58633 | 100.0 |
| 2 | 10.015 | 8.82538 | 24.0 |
| 3 | 11.389 | 7.76302 | 18.0 |
| 4 | 13.469 | 6.56883 | 31.6 |
| 5 | 14.026 | 6.30907 | 14.7 |
| 6 | 17.240 | 5.13927 | 40.6 |
| 7 | 18.814 | 4.71275 | 48.8 |
| 8 | 19.464 | 4.55699 | 61.6 |
| 9 | 20.817 | 4.26363 | 8.8 |
| 10 | 22.483 | 3.95142 | 19.3 |
| 11 | 24.624 | 3.61243 | 14.1 |
| 12 | 26.051 | 3.41776 | 11.9 |

### Example 22. Preparation of Crystal Form V of Sulfate

About 500 mg of compound I was weighed out and suspended in 20 mL of isopropyl acetate, and 109 mg of sulfuric acid was added with stirring at room temperature. The mixture was stirred for 16 h and filtered, and the filter cake was collected and dried *in vacuo* at 60 °C for 4 h to give a product.

### Example 23. Preparation of Crystal Form VI of Sulfate

15 mg of the crystal form I of the sulfate of compound I was weighed out and dispersed in 1 mL of isopropyl acetate. The dispersion was stirred at room temperature for 48 h and filtered, and the filter cake was collected and dried *in vacuo* at 60 °C for 4 h to give a product.

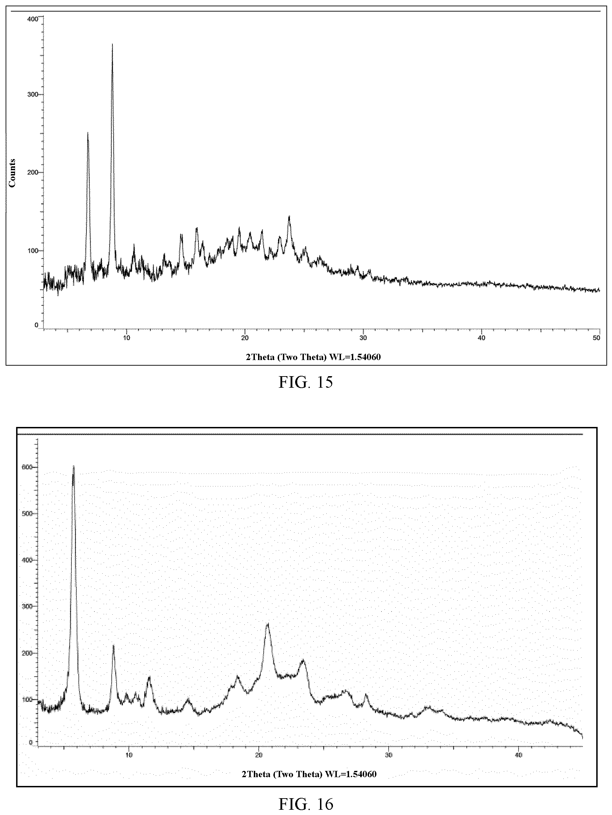

According to an X-ray powder diffraction analysis, the product was defined as the crystal form VI of the sulfate. An XRPD pattern of the crystal form is shown in FIG. 15, and its characteristic peak positions are shown in Table 16.

**Table 16. Peak positions of the crystal form VI of the sulfate**

| Peak No. | 2θ value [° or degrees] | D[Å] | Relative intensity% |
|---|---|---|---|
| 1 | 6.736 | 13.11225 | 61.4 |
| 2 | 8.797 | 10.04362 | 100.0 |
| 3 | 10.619 | 8.32427 | 12.4 |
| 4 | 13.161 | 6.72181 | 8.0 |
| 5 | 14.591 | 6.06609 | 14.5 |
| 6 | 15.948 | 5.55278 | 16.0 |
| 7 | 16.423 | 5.39317 | 8.2 |
| 8 | 18.432 | 4.80964 | 3.3 |
| 9 | 18.951 | 4.67904 | 4.8 |
| 10 | 19.513 | 4.54549 | 9.2 |
| 11 | 20.429 | 4.34383 | 5.8 |
| 12 | 21.387 | 4.15128 | 8.3 |
| 13 | 22.106 | 4.01790 | 1.6 |
| 14 | 22.945 | 3.87291 | 6.4 |
| 15 | 23.708 | 3.74983 | 15.5 |
| 16 | 25.101 | 3.54485 | 4.3 |

### Example 24. Preparation of Crystal Form I of Hydrochloride

About 8 mg of compound I was weighed out and dissolved in 0.2 mL of ethanol, and a hydrochloric acid solution (2 mol/L, 9.8 µL) was added. The mixture was stirred overnight, and 1.0 mL of isopropyl ether was added. The mixture was stirred for crystallization and centrifuged, and the resulting solid was dried *in vacuo* to give a product.

According to an X-ray powder diffraction analysis, the product was defined as the crystal form I of the hydrochloride. An XRPD pattern of the crystal form is shown in FIG. 16, and its characteristic peak positions are shown in Table 17. The chloride ion content was 5.94%, as determined by ion chromatography. A DSC profile shows an exothermic peak at 211.63 °C. A TGA profile shows a weight loss of 5.62% from 30 °C to 175 °C.

**Table 17. Peak positions of the crystal form I of the hydrochloride**

| Peak No. | 2*θ* value [° or degrees] | D[Å] | Relative intensity% |
|---|---|---|---|
| 1 | 5.762 | 15.32493 | 100.0 |
| 2 | 8.814 | 10.02423 | 25.5 |
| 3 | 9.782 | 9.03422 | 5.0 |
| 4 | 10.520 | 8.40208 | 5.3 |
| 5 | 11.558 | 7.64991 | 11.1 |
| 6 | 14.562 | 6.07806 | 4.3 |
| 7 | 18.357 | 4.82908 | 7.2 |
| 8 | 20.664 | 4.29496 | 25.7 |
| 9 | 23.409 | 3.79708 | 13.5 |
| 10 | 26.791 | 3.32491 | 3.9 |
| 11 | 28.162 | 3.16614 | 3.1 |
| 12 | 32.836 | 2.72535 | 1.2 |
| 13 | 34.101 | 2.62708 | 1.6 |

### Example 25. Preparation of Crystal Form II of Hydrochloride

About 8 mg of compound I was weighed out. 0.2 mL of acetonitrile was added, and a hydrochloric acid solution (2 mol/L, 9.8 µL) was added. The mixture was stirred for crystallization and centrifuged, and the resulting solid was dried *in vacuo* to give the title product.

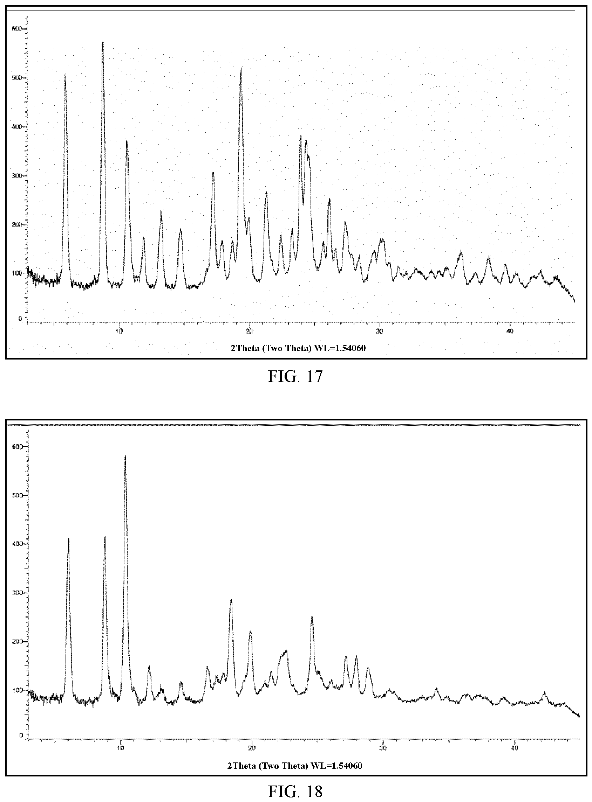

According to an X-ray powder diffraction analysis, the product was defined as the crystal form II of the hydrochloride. An XRPD pattern of the crystal form is shown in FIG. 17, and its characteristic peak positions are shown in Table 18. The chloride ion content was 6.94%, as determined by ion chromatography. A DSC profile shows an endothermic peak at 47.48 °C and an exothermic peak at 221.46 °C. A TGA profile shows a weight loss of 3.32% from 30 °C to 165 °C.

DVS testing shows that the sample had a hygroscopic weight gain of about 3.93% under normal storage conditions (i.e., 25 °C and 60% RH), a hygroscopic weight gain of about 4.28% under accelerated experimental conditions (i.e., 70% RH), and a hygroscopic weight gain of about 5.00% under extreme conditions (i.e., 90% RH). Moreover, according to a crystal form re-identification, the crystal form did not change after DVS testing.

**Table 18. Peak positions of the crystal form II of the hydrochloride**

| Peak No. | 2*θ* value [° or degrees] | D[Å] | Relative intensity (%) |
|---|---|---|---|
| 1 | 5.895 | 14.98026 | 82.7 |
| 2 | 8.759 | 10.08688 | 100.0 |
| 3 | 10.605 | 8.33492 | 58.2 |
| 4 | 11.889 | 7.43780 | 19.0 |
| 5 | 13.213 | 6.69529 | 30.7 |
| 6 | 14.720 | 6.01302 | 23.1 |
| 7 | 17.229 | 5.14267 | 43.7 |
| 8 | 17.913 | 4.94785 | 14.4 |
| 9 | 18.711 | 4.73843 | 13.0 |
| 10 | 19.346 | 4.58449 | 85.0 |
| 11 | 19.947 | 4.44756 | 23.6 |
| 12 | 21.294 | 4.16924 | 34.0 |
| 13 | 22.413 | 3.96352 | 14.4 |
| 14 | 23.275 | 3.81868 | 16.3 |
| 15 | 23.937 | 3.71460 | 53.8 |
| 16 | 24.363 | 3.65050 | 51.5 |
| 17 | 24.519 | 3.62763 | 44.6 |
| 18 | 25.664 | 3.46837 | 10.0 |
| 19 | 26.125 | 3.40821 | 25.9 |
| 20 | 26.611 | 3.34707 | 7.9 |
| 21 | 27.362 | 3.25689 | 20.1 |
| 22 | 27.846 | 3.20138 | 7.3 |
| 23 | 28.418 | 3.13814 | 8.5 |
| 24 | 29.584 | 3.01708 | 10.5 |
| 25 | 30.250 | 2.95216 | 15.1 |
| 26 | 30.762 | 2.90415 | 5.5 |
| 27 | 31.430 | 2.84396 | 3.6 |
| 28 | 32.028 | 2.79226 | 0.7 |
| 29 | 32.836 | 2.72535 | 2.2 |
| 30 | 33.960 | 2.63763 | 2.4 |
| 31 | 34.488 | 2.59852 | 1.2 |
| 32 | 35.155 | 2.55068 | 3.0 |
| 33 | 36.238 | 2.47694 | 11.0 |
| 34 | 37.369 | 2.40449 | 3.0 |
| 35 | 38.378 | 2.34356 | 9.1 |
| 36 | 39.623 | 2.27276 | 5.8 |
| 37 | 40.462 | 2.22756 | 2.7 |
| 38 | 41.692 | 2.16464 | 2.8 |
| 39 | 42.359 | 2.13205 | 4.8 |
| 40 | 43.484 | 2.07948 | 4.3 |

### Example 26. Preparation of Crystal Form II of Hydrochloride

Compound I was weighed out, and a solvent and a 2 mol/L hydrochloric acid solution were added. Crystallization was performed to give a product. The crystal form was determined by X-ray powder diffraction, as shown in Table 19.

**Table 19. Preparation of the crystal form II of the hydrochloride**

| No. | Feed amount and solvent | Crystal form |
|---|---|---|
| 1 | About 8 mg of compound I was weighed out. 0.2 mL of isopropanol was added, and hydrochloric acid (2 mol/L, 9.8 µL) was added. The mixture was stirred for crystallization. | Crystal form II of hydrochloride |
| 2 | About 120 mg of compound I was weighed out and dissolved in 0.8 mL of ethanol. Hydrochloric acid (2 mol/L, 140 µL) was added, and 2 mL of isopropyl ether was added. The mixture was stirred for crystallization. | Crystal form II of hydrochloride |

### Example 27. Preparation of Crystal Form III of Hydrochloride

About 8 mg of compound I was weighed out. 0.2 mL of acetone was added, and a hydrochloric acid solution (2 mol/L, 9.8 µL) was added. The mixture was stirred for crystallization and centrifuged, and the resulting solid was dried *in vacuo* to give a product.

According to an X-ray powder diffraction analysis, the product was defined as the crystal form III of the hydrochloride. An XRPD pattern of the crystal form is shown in FIG. 18, and its characteristic peak positions are shown in Table 20. The chloride ion content was 6.68%, as determined by ion chromatography. A DSC profile shows an exothermic peak at 191.78 °C and an endothermic peak at 206.27 °C. A TGA profile shows a weight loss of 2.34% from 30 °C to 155 °C.

DVS testing shows that the sample had a hygroscopic weight gain of about 2.57% under normal storage conditions (i.e., 25 °C and 60% RH), a hygroscopic weight gain of about 2.96% under accelerated experimental conditions (i.e., 70% RH), and a hygroscopic weight gain of about 4.57% under extreme conditions (i.e., 90% RH). Moreover, according to a crystal form re-identification, the crystal form did not change after DVS testing.

**Table 20. Peak positions of the crystal form III of the hydrochloride**

| Peak No. | 2*θ* value [° or degrees] | D[Å] | Relative intensity (%) |
|---|---|---|---|
| 1 | 6.051 | 14.59539 | 65.8 |
| 2 | 8.822 | 10.01598 | 66.9 |
| 3 | 10.379 | 8.51589 | 100.0 |
| 4 | 12.186 | 7.25726 | 13.7 |
| 5 | 13.086 | 6.76011 | 3.4 |
| 6 | 14.613 | 6.05686 | 7.4 |
| 7 | 16.620 | 5.32983 | 12.3 |
| 8 | 17.338 | 5.11056 | 6.7 |
| 9 | 17.830 | 4.97064 | 7.8 |
| 10 | 18.427 | 4.81107 | 37.7 |
| 11 | 19.901 | 4.45772 | 23.4 |
| 12 | 20.993 | 4.22835 | 2.6 |
| 13 | 21.467 | 4.13598 | 6.5 |
| 14 | 22.246 | 3.99285 | 13.5 |
| 15 | 22.564 | 3.93743 | 15.2 |
| 16 | 24.579 | 3.61897 | 28.8 |
| 17 | 26.089 | 3.41287 | 1.7 |
| 18 | 27.158 | 3.28086 | 11.9 |
| 19 | 27.966 | 3.18791 | 12.8 |
| 20 | 28.863 | 3.09083 | 9.5 |
| 21 | 30.481 | 2.93030 | 1.5 |
| 22 | 33.996 | 2.63499 | 2.1 |
| 23 | 36.174 | 2.48112 | 2.1 |
| 24 | 39.232 | 2.29452 | 0.9 |
| 25 | 42.254 | 2.13713 | 3.9 |

### Example 28. Preparation of Crystal Form III of Hydrochloride

About 120 mg of compound I was weighed out. 3 mL of ethyl acetate was added, and a solution of hydrochloric acid in ethanol (2 mol/L, 140 µL) was added. The mixture was stirred for crystallization and centrifuged, and the resulting solid was dried *in vacuo* to give a product.

The product was the crystal form III of the hydrochloride, as identified by X-ray powder diffraction.

### Example 29. Preparation of Crystal Form IV of Hydrochloride

About 8 mg of compound I was weighed out and dissolved in 0.2 mL of tetrahydrofuran, and a hydrochloric acid solution (2 mol/L, 9.8 µL) was added. The mixture was stirred for crystallization and centrifuged, and the resulting solid was dried *in vacuo* to give a product.

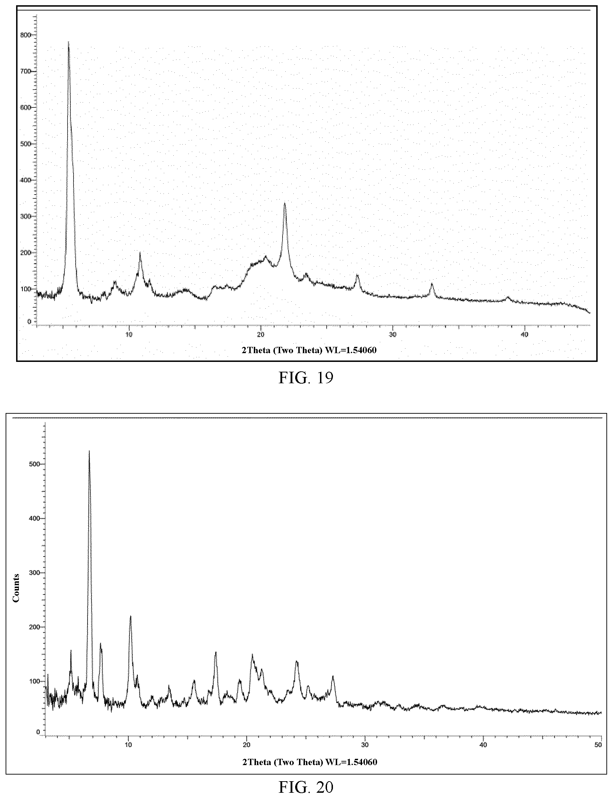

According to an X-ray powder diffraction analysis, the product was defined as the crystal form IV of the hydrochloride. An XRPD pattern of the crystal form is shown in FIG. 19, and its characteristic peak positions are shown in Table 21. A DSC profile shows an exothermic peak at 208.82 °C. A TGA profile shows a weight loss of 2.60% from 30 °C to 160 °C.

**Table 21. Peak positions of the crystal form IV of the hydrochloride**

| Peak No. | 2*θ* value [° or degrees] | D[Å] | Relative intensity (%) |
|---|---|---|---|
| 1 | 5.423 | 16.28430 | 100.0 |
| 2 | 8.096 | 10.91244 | 0.8 |
| 3 | 8.998 | 9.82034 | 5.4 |
| 4 | 10.848 | 8.14940 | 15.2 |
| 5 | 11.575 | 7.63905 | 4.8 |
| 6 | 14.351 | 6.16688 | 2.1 |
| 7 | 16.424 | 5.39275 | 2.4 |
| 8 | 19.306 | 4.59383 | 5.5 |
| 9 | 20.395 | 4.35084 | 7.4 |
| 10 | 21.814 | 4.07106 | 28.5 |
| 11 | 23.453 | 3.79011 | 1.9 |
| 12 | 27.316 | 3.26228 | 4.9 |
| 13 | 32.965 | 2.71500 | 4.5 |
| 14 | 38.775 | 2.32049 | 1.0 |

### Example 30. Preparation of Crystal Form V of Hydrochloride

About 140 mg of compound I was weighed out and dissolved in 5 mL of acetonitrile, and 33 mg of concentrated hydrochloric acid was added with stirring. The mixture was stirred for 16 h and filtered, and the filter cake was collected and dried *in vacuo* at 60 °C for 4 h to give a product.

According to an X-ray powder diffraction analysis, the product was defined as the crystal form V of the hydrochloride. An XRPD pattern of the crystal form is shown in FIG. 20, and its characteristic peak positions are shown in Table 22.

A DSC profile shows an endothermic peak at 63.15 °C and exothermic peaks at 196.81 °C and 211.82 °C. A TGA profile shows a weight loss of 4.60% from 30 °C to 170 °C and a weight loss of 6.81% from 170 °C to 260 °C. DVS data show that the sample had a hygroscopic weight gain of about 6.36% under normal storage conditions (i.e., 25 °C and 60% RH), a hygroscopic weight gain of about 7.28% under accelerated test conditions (i.e., 70% RH), and a hygroscopic weight gain of about 8.32% under extreme conditions (90% RH). The XRPD pattern shows that the crystal form of the sample did not change before or after DVS.

**Table 22. XRPD data for the crystal form V of the hydrochloride**

| Peak No. | 2θ value [° or degrees] | D[Å] | Relative intensity (%) |
|---|---|---|---|
| 1 | 5.158 | 17.12050 | 20.60 |
| 2 | 6.707 | 13.16750 | 100.00 |
| 3 | 7.666 | 11.52280 | 23.80 |
| 4 | 10.190 | 8.67390 | 35.50 |
| 5 | 10.760 | 8.21570 | 11.80 |
| 6 | 13.418 | 6.59370 | 7.30 |
| 7 | 15.577 | 5.68410 | 7.40 |
| 8 | 17.376 | 5.09940 | 19.40 |
| 9 | 19.388 | 4.57463 | 7.60 |
| 10 | 20.489 | 4.33128 | 17.00 |
| 11 | 21.273 | 4.17331 | 11.00 |
| 12 | 24.218 | 3.67204 | 13.80 |
| 13 | 27.288 | 3.26555 | 9.60 |

### Example 31. Preparation of Crystal Form VI of Hydrochloride

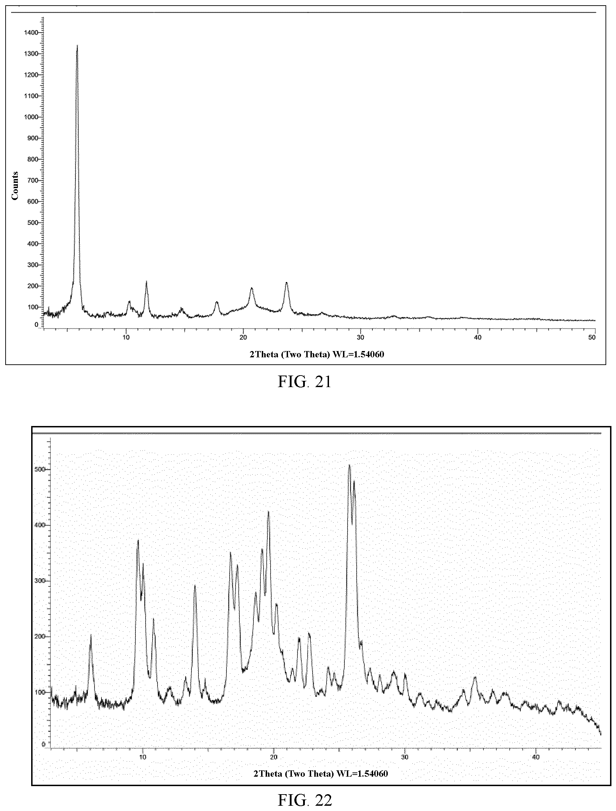

About 140 mg of compound I was weighed out and dissolved in 5 mL of isopropanol, and 78 µL of a 4 M solution of hydrochloric acid in dioxane was added with stirring. The mixture was stirred for 16 h and filtered, and the filter cake was collected and dried *in vacuo* at 60 °C for 4 h to give a product. According to an X-ray powder diffraction analysis, the product was defined as the crystal form VI of the hydrochloride. An XRPD pattern of the crystal form is shown in FIG. 21, and its characteristic peak positions are shown in Table 23. ADSC profile shows exothermic peaks at 104.31 °C, 198.49 °C, and 204.67 °C. A TGA profile shows a weight loss of 3.22% from 30 °C to 195 °C and a weight loss of 4.49% from 195 °C to 265 °C. DVS data show that the sample had a hygroscopic weight gain of about 3.69% under normal storage conditions (i.e., 25 °C and 60% RH), a hygroscopic weight gain of about 4.12% under accelerated test conditions (i.e., 70% RH), and a hygroscopic weight gain of about 5.73% under extreme conditions (90% RH). The XRPD pattern shows that the crystal form of the sample did not change before or after DVS.

**Table 23. XRPD data for the crystal form VI of the hydrochloride**

| Peak No. | 2θ value [° or degrees] | D[Å] | Relative intensity% |
|---|---|---|---|
| 1 | 5.845 | 15.10932 | 100.00 |
| 2 | 10.253 | 8.62101 | 5.00 |
| 3 | 11.735 | 7.53482 | 12.90 |
| 4 | 14.760 | 5.99691 | 1.90 |
| 5 | 17.727 | 4.99918 | 4.70 |
| 6 | 20.716 | 4.28418 | 7.60 |
| 7 | 23.654 | 3.75826 | 10.80 |

### Example 32. Preparation of Crystal Form I of Fumarate

About 8 mg of compound I and about 2.27 mg of fumaric acid were weighed out, and 0.2 mL of acetonitrile was added. The mixture was stirred for crystallization and centrifuged, and the resulting solid was dried *in vacuo* to give a product.

According to an X-ray powder diffraction analysis, the product was defined as the crystal form I of the fumarate. An XRPD pattern of the crystal form is shown in FIG. 22, and its characteristic peak positions are shown in Table 24. The fumarate ion content was 20.34%, as determined by ion chromatography. A DSC profile shows an endothermic peak at 179.08 °C. A TGA profile shows a weight loss of 1.02% from 30 °C to 150 °C.

DVS testing shows that the sample had a hygroscopic weight gain of about 0.51% under normal storage conditions (i.e., 25 °C and 60% RH), a hygroscopic weight gain of about 0.60% under accelerated experimental conditions (i.e., 70% RH), and a hygroscopic weight gain of about 0.82% under extreme conditions (i.e., 90% RH). Moreover, according to a crystal form re-identification, the crystal form did not change after DVS testing.

**Table 24. Peak positions of the crystal form I of the fumarate**

| Peak No. | 2*θ* value [° or degrees] | D[Å] | Relative intensity% |
|---|---|---|---|
| 1 | 4.898 | 18.02822 | 7.7 |
| 2 | 6.057 | 14.58063 | 31.1 |
| 3 | 9.634 | 9.17322 | 72.7 |
| 4 | 10.031 | 8.81136 | 62.2 |
| 5 | 10.838 | 8.15644 | 36.4 |
| 6 | 12.067 | 7.32867 | 5.1 |
| 7 | 13.275 | 6.66409 | 10.1 |
| 8 | 14.003 | 6.31931 | 51.8 |
| 9 | 14.781 | 5.98853 | 8.9 |
| 10 | 16.733 | 5.29395 | 62.1 |
| 11 | 17.225 | 5.14381 | 55.8 |
| 12 | 18.635 | 4.75775 | 40.0 |
| 13 | 19.119 | 4.63834 | 59.5 |
| 14 | 19.612 | 4.52276 | 76.2 |
| 15 | 20.212 | 4.38986 | 33.0 |
| 16 | 21.415 | 4.14603 | 5.1 |
| 17 | 21.965 | 4.04345 | 20.1 |
| 18 | 22.699 | 3.91418 | 24.6 |
| 19 | 24.171 | 3.67905 | 10.1 |
| 20 | 24.648 | 3.60902 | 6.4 |
| 21 | 25.799 | 3.45048 | 100.0 |
| 22 | 26.137 | 3.40663 | 93.3 |
| 23 | 26.639 | 3.34354 | 18.1 |
| 24 | 27.351 | 3.25814 | 7.6 |
| 25 | 28.092 | 3.17390 | 5.2 |
| 26 | 29.149 | 3.06117 | 9.1 |
| 27 | 30.041 | 2.97225 | 10.2 |
| 28 | 31.219 | 2.86269 | 4.7 |
| 29 | 31.852 | 2.80727 | 1.9 |
| 30 | 32.449 | 2.75693 | 1.6 |
| 31 | 34.523 | 2.59595 | 4.8 |
| 32 | 35.364 | 2.53613 | 10.0 |
| 33 | 36.737 | 2.44443 | 3.1 |
| 34 | 37.545 | 2.39364 | 2.6 |
| 35 | 39.197 | 2.29649 | 2.1 |
| 36 | 40.743 | 2.21283 | 2.1 |
| 37 | 41.797 | 2.15942 | 4.9 |
| 38 | 43.062 | 2.09887 | 5.1 |

### Example 33. Preparation of Crystal Form I of Fumarate

About 80 mg of compound I was weighed out and added to a solution of fumaric acid in methanol (0.33 mol/L, 537 µL), and 1.5 mL of acetonitrile was added. The mixture was stirred for crystallization and centrifuged, and the resulting solid was dried *in vacuo* to give a product.

The product was the crystal form I of the fumarate, as identified by X-ray powder diffraction.

### Example 34. Preparation of Crystal Form II of Fumarate

About 8 mg of compound I and 2.27 mg of fumaric acid were weighed out and dissolved in 0.2 mL of methanol/acetonitrile (V/V = 1:8). The mixture was filtered and volatilized for crystallization to give a product.

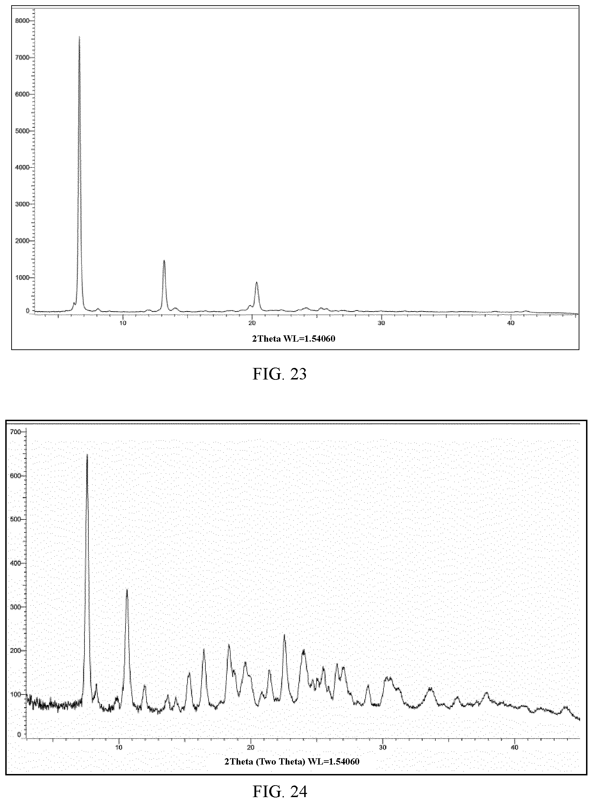

According to an X-ray powder diffraction analysis, the product was defined as the crystal form II of the fumarate. An XRPD pattern of the crystal form is shown in FIG. 23, and its characteristic peak positions are shown in Table 25.

**Table 25. Peak positions of the crystal form II of the fumarate**

| Peak No. | 2*θ* value [° or degrees] | D[Å] | Relative intensity% |
|---|---|---|---|
| 1 | 6.233 | 14.16954 | 3.0 |
| 2 | 6.638 | 13.30424 | 100.0 |
| 3 | 8.083 | 10.92947 | 1.0 |
| 4 | 8.981 | 9.83854 | 0.2 |
| 5 | 11.985 | 7.37867 | 0.4 |
| 6 | 13.199 | 6.70264 | 18.5 |
| 7 | 14.030 | 6.30714 | 1.2 |
| 8 | 16.410 | 5.39734 | 0.2 |
| 9 | 17.108 | 5.17880 | 0.1 |
| 10 | 18.085 | 4.90127 | 0.2 |
| 11 | 18.503 | 4.79133 | 0.2 |
| 12 | 19.282 | 4.59960 | 0.4 |
| 13 | 19.842 | 4.47104 | 1.8 |
| 14 | 20.349 | 4.36071 | 10.4 |
| 15 | 21.398 | 4.14920 | 0.1 |
| 16 | 21.852 | 4.06411 | 0.2 |
| 17 | 22.260 | 3.99053 | 0.4 |
| 18 | 23.623 | 3.76322 | 0.5 |
| 19 | 24.191 | 3.67606 | 1.1 |
| 20 | 25.335 | 3.51262 | 1.0 |
| 21 | 25.788 | 3.45198 | 0.8 |
| 22 | 26.874 | 3.31486 | 0.2 |
| 23 | 27.223 | 3.27318 | 0.2 |
| 24 | 28.130 | 3.16968 | 0.3 |
| 25 | 29.978 | 2.97830 | 0.2 |
| 26 | 31.897 | 2.80342 | 0.2 |
| 27 | 41.150 | 2.19186 | 0.4 |

### Example 35. Preparation of Crystal Form I of Hydrobromide

About 8 mg of compound I was weighed out and dissolved in 0.2 mL of ethanol, and a hydrobromic acid solution (2 mol/L, 9.8 µL) was added. The mixture was stirred overnight, and 0.8 mL of isopropyl ether was added. The mixture was stirred for crystallization and centrifuged, and the resulting solid was dried *in vacuo* to give a product.

According to an X-ray powder diffraction analysis, the product was defined as the crystal form I of the hydrobromide. An XRPD pattern of the crystal form is shown in FIG. 24, and its characteristic peak positions are shown in Table 26. The bromide ion content was 13.98%, as determined by ion chromatography. A DSC profile shows an exothermic peak at 216.67 °C. A TGA profile shows a weight loss of 1.24% from 30 °C to 160 °C.

**Table 26. Peak positions of the crystal form I of the hydrobromide**

| Peak No. | 2*θ* value [° or degrees] | D[Å] | Relative intensity (%) |
|---|---|---|---|
| 1 | 7.597 | 11.62745 | 100.0 |
| 2 | 8.299 | 10.64596 | 8.5 |
| 3 | 9.878 | 8.94727 | 4.6 |
| 4 | 10.620 | 8.32360 | 47.4 |
| 5 | 11.978 | 7.38259 | 9.0 |
| 6 | 13.718 | 6.45020 | 5.9 |
| 7 | 14.324 | 6.17842 | 4.6 |
| 8 | 15.349 | 5.76815 | 14.1 |
| 9 | 16.448 | 5.38503 | 22.6 |
| 10 | 18.350 | 4.83090 | 22.9 |
| 11 | 18.698 | 4.74195 | 12.1 |
| 12 | 19.583 | 4.52940 | 15.3 |
| 13 | 19.869 | 4.46503 | 10.4 |
| 14 | 20.837 | 4.25954 | 4.1 |
| 15 | 21.398 | 4.14914 | 12.0 |
| 16 | 22.557 | 3.93861 | 25.7 |
| 17 | 24.008 | 3.70370 | 18.4 |
| 18 | 24.718 | 3.59892 | 6.1 |
| 19 | 25.034 | 3.55416 | 5.9 |
| 20 | 25.488 | 3.49197 | 11.0 |
| 21 | 26.534 | 3.35655 | 12.8 |
| 22 | 27.043 | 3.29450 | 12.4 |
| 23 | 28.887 | 3.08825 | 6.5 |
| 24 | 30.295 | 2.94786 | 9.6 |
| 25 | 30.657 | 2.91390 | 8.9 |
| 26 | 31.149 | 2.86899 | 5.6 |
| 27 | 33.610 | 2.66435 | 5.5 |
| 28 | 35.647 | 2.51659 | 2.8 |
| 29 | 37.826 | 2.37649 | 3.5 |
| 30 | 43.941 | 2.05892 | 1.9 |

### Example 36. Preparation of Crystal Form II of Hydrobromide

About 8 mg of compound I was weighed out. 0.2 mL of isopropanol was added, and a solution of hydrobromic acid in ethanol (2 mol/L, 9.8 µL) was added. The mixture was stirred for crystallization and centrifuged, and the resulting solid was dried *in vacuo* to give the title product.

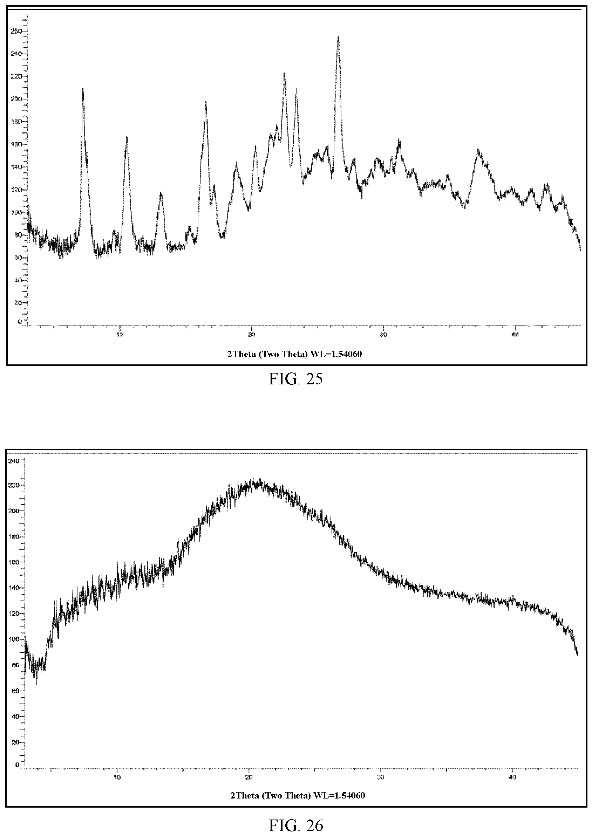

According to an X-ray powder diffraction analysis, the product was defined as the crystal form II of the hydrobromide. An XRPD pattern of the crystal form is shown in FIG. 25, and its characteristic peak positions are shown in Table 27. The bromide ion content was 14.89%, as determined by ion chromatography. A DSC profile shows an exothermic peak at 211.47 °C. A TGA profile shows a weight loss of 0.77% from 30 °C to 145 °C.

**Table 27. Peak positions of the crystal form II of the hydrobromide**

| Peak No. | 2*θ* value [° or degrees] | D[Å] | Relative intensity (%) |
|---|---|---|---|
| 1 | 7.232 | 12.21399 | 100.0 |
| 2 | 10.544 | 8.38361 | 68.2 |
| 3 | 13.131 | 6.73688 | 32.0 |
| 4 | 16.504 | 5.36706 | 77.5 |
| 5 | 17.174 | 5.15899 | 29.5 |
| 6 | 18.847 | 4.70475 | 33.3 |
| 7 | 20.298 | 4.37160 | 31.9 |
| 8 | 21.485 | 4.13263 | 32.0 |
| 9 | 21.871 | 4.06045 | 35.9 |
| 10 | 22.486 | 3.95079 | 67.3 |
| 11 | 23.401 | 3.79845 | 56.5 |
| 12 | 25.069 | 3.54926 | 12.3 |
| 13 | 25.737 | 3.45867 | 11.1 |
| 14 | 26.578 | 3.35115 | 90.0 |
| 15 | 27.811 | 3.20534 | 11.7 |
| 16 | 29.076 | 3.06869 | 8.0 |
| 17 | 29.568 | 3.01873 | 12.9 |
| 18 | 30.657 | 2.91390 | 9.8 |
| 19 | 31.290 | 2.85642 | 20.5 |
| 20 | 32.309 | 2.76860 | 7.8 |
| 21 | 34.804 | 2.57562 | 4.4 |
| 22 | 37.238 | 2.41263 | 23.8 |
| 23 | 37.896 | 2.37224 | 14.8 |
| 24 | 39.794 | 2.26338 | 0.5 |
| 25 | 41.270 | 2.18578 | 4.3 |
| 26 | 42.465 | 2.12700 | 14.0 |
| 27 | 43.554 | 2.07629 | 13.2 |

### Example 37. Preparation of Crystal Form II of Hydrobromide

About 8 mg of compound I was weighed out. 0.2 mL of ethyl acetate was added, and a solution of hydrobromic acid in ethanol (2 mol/L, 9.8 µL) was added. The mixture was stirred for crystallization and centrifuged, and the resulting solid was dried *in vacuo* to give the title product.

The product was the crystal form II of the hydrobromide, as identified by X-ray powder diffraction.

### Example 38. Preparation of Amorphous Form of Maleate

About 8 mg of compound I was weighed out and dissolved in 0.2 mL of ethanol, and a maleic acid solution (2 mol/L, 9.8 µL) was added. The compound was slurried overnight at room temperature, and 0.6 mL of isopropyl ether was added. The mixture was stirred for precipitation and centrifuged, and the resulting solid was dried *in vacuo* to give a product.

The product was the amorphous form of the maleate, as identified by X-ray powder diffraction.

### Example 39. Preparation of Amorphous Form of Maleate

Compound I was weighed out, and a solvent and a 2 mol/L maleic acid solution were added. Crystallization was performed to give a product. The crystal form was determined by X-ray powder diffraction, as shown in Table 28.

**Table 28. Preparation of the amorphous form of the maleate**

| No. | Feed amount and solvent | Crystal form |
|---|---|---|
| 1 | About 8 mg of compound I was weighed out and dissolved in 0.2 mL of acetone, and a maleic acid solution (2 mol/L, 9.8 µL) was added. The compound was slurried overnight at room temperature, and 0.6 mL of isopropyl ether was added. The mixture was stirred for precipitation. | Amorphous form of maleate |
| 2 | About 8 mg of compound I was weighed out and dissolved in 0.2 mL of tetrahydrofuran, and a maleic acid solution (2 mol/L, 9.8 µL) was added. The compound was slurried overnight at room temperature, and 0.6 mL of isopropyl ether was added. The mixture was stirred for precipitation. | Amorphous form of maleate |
| 3 | About 8 mg of compound I was weighed out and dissolved in 0.2 mL of acetonitrile/methanol (V/V = 1:1), and a maleic acid solution (2 mol/L, 9.8 µL) was added. The compound was slurried overnight at room temperature, and 0.6 mL of isopropyl ether was added. The mixture was stirred for precipitation. | Amorphous form of maleate |
| 4 | About 8 mg of compound I was weighed out. 0.2 mL of isopropanol was added, and a solution of maleic acid in ethanol (2 mol/L, 9.8 µL) was added. The compound was slurried overnight at room temperature, and 0.6 mL of isopropyl ether was added. The mixture was stirred for precipitation. | Amorphous form of maleate |
| 5 | About 8 mg of compound I was weighed out. 0.2 mL of ethyl acetate was added, and a solution of maleic acid in ethanol (2 mol/L, 9.8 µL) was added. The compound was slurried overnight at room temperature, and 0.6 mL of isopropyl ether was added. The mixture was stirred for precipitation. | Amorphous form of maleate |

### Example 40. Preparation of Amorphous Form of Phosphate

About 8 mg of compound I was weighed out and dissolved in 0.2 mL of ethanol, and a phosphoric acid solution (2 mol/L, 9.8 µL) was added. The mixture was stirred for precipitation and centrifuged, and the resulting solid was dried *in vacuo* to give a product.

The product was the amorphous form of the phosphate, as identified by X-ray powder diffraction.

### Example 41. Preparation of Amorphous Form of Phosphate

Compound I was weighed out, and a solvent and a 2 mol/L phosphoric acid solution were added. Crystallization was performed to give a product. The crystal form was determined by X-ray powder diffraction, as shown in Table 29.

**Table 29. Preparation of the amorphous form of the phosphate**

| No. | Feed amount and solvent | Crystal form |
|---|---|---|
| 1 | About 8 mg of compound I was weighed out and dissolved in 0.2 mL of tetrahydrofuran, and phosphoric acid (2 mol/L, 9.8 µL) was added. The mixture was stirred for precipitation. | Amorphous form of phosphate |
| 2 | About 8 mg of compound I was weighed out and dissolved in 0.2 mL of acetonitrile/methanol (V/V = 1:1), and a solution of phosphoric acid in ethanol (2 mol/L, 9.8 µL) was added. The compound was slurried overnight at room temperature, and 0.6 mL of isopropyl ether was added. The mixture was stirred for precipitation. | Amorphous form of phosphate |

### Example 42. Preparation of Amorphous Form of p-Toluenesulfonate

About 8 mg of compound I was weighed out. 0.2 mL of acetonitrile was added, and a p-toluenesulfonic acid solution (2 mol/L, 9.8 µL) was added. The compound was slurried overnight at room temperature, and 0.6 mL of isopropyl ether was added. The mixture was stirred for precipitation and centrifuged, and the resulting solid was dried *in vacuo* to give a product.

The product was the amorphous form of the p-toluenesulfonate, as identified by X-ray powder diffraction.

### Example 43. Preparation of Amorphous Form of p-Toluenesulfonate

Compound I was weighed out, and a solvent and a 2 mol/L p-toluenesulfonic acid solution were added. Crystallization was performed to give a product. The crystal form was determined by X-ray powder diffraction, as shown in Table 30.

**Table 30. Preparation of the amorphous form of the p-toluenesulfonate**

| No. | Feed amount and solvent | Crystal form |
|---|---|---|
| 1 | About 8 mg of compound I was weighed out. 0.2 mL of acetone was added, and a p-toluenesulfonic acid solution (2 mol/L, 9.8 µL) was added. The compound was slurried overnight at room temperature, and 0.6 mL of isopropyl ether was added. The mixture was stirred for precipitation. | Amorphous form of p-toluenesulfonate |
| 2 | About 8 mg of compound I was weighed out. 0.2 mL of tetrahydrofuran was added, and a p-toluenesulfonic acid solution (2 mol/L, 9.8 µL) was added. The compound was slurried overnight at room temperature, and 0.6 mL of isopropyl ether was added. The mixture was stirred for precipitation. | Amorphous form of p-toluenesulfonate |
| 3 | About 8 mg of compound I was weighed out. 0.2 mL of acetonitrile/methanol (V/V = 1: 1) was added, and a p-toluenesulfonic acid solution (2 mol/L, 9.8 µL) was added. The compound was slurried overnight at room temperature, and 0.6 mL of isopropyl ether was added. The mixture was stirred for precipitation. | Amorphous form of p-toluenesulfonate |

### Example 44. Preparation of Amorphous Form of Sulfate

About 8 mg of compound I was weighed out and dissolved in 0.2 mL of tetrahydrofuran, and a sulfuric acid solution (2 mol/L, 9.8 µL) was added. The compound was slurried overnight at room temperature. The mixture was stirred for precipitation and centrifuged, and the resulting solid was dried *in vacuo* to give a product.

The product was the amorphous form of the sulfate, as identified by X-ray powder diffraction.

### Example 45. Preparation of Amorphous Form of Sulfate

About 8 mg of compound I was weighed out and dissolved in 0.2 mL of acetonitrile/methanol (V/V = 1:1), and a sulfuric acid solution (2 mol/L, 9.8 µL) was added. The compound was slurried overnight at room temperature, and 1 mL of isopropyl ether was added. The mixture was stirred for precipitation and centrifuged, and the resulting solid was dried *in vacuo* to give a product.

The product was the amorphous form of the sulfate, as identified by X-ray powder diffraction.

### Example 46. Preparation of Amorphous Form of Tartrate

About 8 mg of compound I was weighed out. 0.2 mL of acetonitrile was added, and a tartaric acid solution (2 mol/L, 9.8 µL) was added. The mixture was stirred for precipitation and centrifuged, and the resulting solid was dried *in vacuo* to give a product.

The product was the amorphous form of the tartrate, as identified by X-ray powder diffraction.

### Example 47. Preparation of Amorphous Form of Tartrate

Compound I was weighed out, and a solvent and a 2 mol/L tartaric acid solution were added. Crystallization was performed to give a product. The crystal form was determined by X-ray powder diffraction, as shown in Table 31.

**Table 31. Preparation of the amorphous form of the tartrate**

| No. | Feed amount and solvent | Crystal form |
|---|---|---|
| 1 | About 8 mg of compound I was weighed out. 0.2 mL of acetone was added, and a tartaric acid solution (2 mol/L, 9.8 µL) was added. The mixture was stirred for precipitation. | Amorphous form of tartrate |
| 2 | About 8 mg of compound I was weighed out and dissolved in 0.2 mL of ethanol, and a tartaric acid solution (2 mol/L, 9.8 µL) was added. The compound was slurried overnight at room temperature, and 0.8 mL of isopropyl ether was added. The mixture was stirred for precipitation. | Amorphous form of tartrate |
| 3 | About 8 mg of compound I was weighed out and dissolved in 0.2 mL of tetrahydrofuran, and a tartaric acid solution (2 mol/L, 9.8 µL) was added. The compound was slurried overnight at room temperature, and 0.8 mL of isopropyl ether was added. The mixture was stirred for precipitation. | Amorphous form of tartrate |

### Example 48. Preparation of Amorphous Form of Succinate

About 8 mg of compound I and about 2.3 mg of succinic acid were weighed out, and 0.2 mL of acetonitrile was added. The compound was slurried overnight at room temperature. The mixture was centrifuged, and the resulting solid was dried *in vacuo* to give a product.

The product was the amorphous form of the succinate, as identified by X-ray powder diffraction.

### Example 49. Preparation of Amorphous Form of Succinate

Compound I was weighed out, and a solvent and succinic acid were added. Crystallization was performed to give a product. The crystal form was determined by X-ray powder diffraction, as shown in Table 32.

**Table 32. Preparation of the amorphous form of the succinate**

| No. | Feed amount and solvent | Crystal form |
|---|---|---|
| 1 | About 8 mg of compound I and about 2.3 mg of succinic acid were weighed out, and 0.2 mL of acetone was added. The compound was slurried overnight at room temperature. | Amorphous form of succinate |
| 2 | About 8 mg of compound I and about 2.3 mg of succinic acid were weighed out, and 0.2 mL of ethanol was added. The compound was slurried overnight at room temperature, and 0.6 mL of isopropyl ether was added. The mixture was stirred for precipitation. | Amorphous form of succinate |
| 3 | About 8 mg of compound I and about 2.3 mg of succinic acid were weighed out, and 0.2 mL of tetrahydrofuran was added. The compound was slurried overnight at room temperature, and 0.6 mL of isopropyl ether was added. The mixture was stirred for precipitation. | Amorphous form of succinate |

### Example 50. Preparation of Amorphous Form of Fumarate

About 8 mg of compound I and about 2.3 mg of fumaric acid were weighed out, and 0.2 mL of acetone was added. The compound was slurried overnight at room temperature. The mixture was centrifuged, and the resulting solid was dried *in vacuo* to give a product.

The product was the amorphous form of the fumarate, as identified by X-ray powder diffraction. An XRPD pattern of the amorphous form is shown in FIG. 26.

### Example 51. Preparation of Amorphous Form of Fumarate

Compound I was weighed out, and a solvent and fumaric acid were added. Crystallization was performed to give a product. The crystal form was determined by X-ray powder diffraction, as shown in Table 33.

**Table 33. Preparation of the amorphous form of the fumarate**

| No. | Feed amount and solvent | Crystal form |
|---|---|---|
| 1 | About 8 mg of compound I and about 2.3 mg of fumaric acid were weighed out and dissolved in 0.2 mL of ethanol. The compound was slurried overnight at room temperature, and 0.6 mL of isopropyl ether was added. The mixture was stirred for precipitation. | Amorphous form of fumarate |
| 2 | About 8 mg of compound I and about 2.3 mg of fumaric acid were weighed out and dissolved in 0.2 mL of tetrahydrofuran. The compound was slurried overnight at room temperature, and 0.6 mL of isopropyl ether was added. The mixture was stirred for precipitation. | Amorphous form of fumarate |

### Example 52. Preparation of Amorphous Form of Citrate

About 8 mg of compound I was weighed out and dissolved in 0.2 mL of ethanol, and a citric acid solution (2 mol/L, 9.8 µL) was added. The compound was slurried overnight at room temperature, and 0.6 mL of isopropyl ether was added. The mixture was stirred for precipitation and centrifuged, and the resulting solid was dried *in vacuo* to give a product.

The product was the amorphous form of the citrate, as identified by X-ray powder diffraction.

### Example 53. Preparation of Amorphous Form of Citrate

Compound I was weighed out, and a solvent and a 2 mol/L citric acid solution were added. Crystallization was performed to give a product. The crystal form was determined by X-ray powder diffraction, as shown in Table 34.

**Table 34. Preparation of the amorphous form of the citrate**

| No. | Feed amount and solvent | Crystal form |
|---|---|---|
| 1 | About 8 mg of compound I was weighed out and dissolved in 0.2 mL of acetonitrile, and a citric acid solution (2 mol/L, 9.8 µL) was added. The compound was slurried overnight at room temperature, and 0.6 mL of isopropyl ether was added. The mixture was stirred for precipitation. | Amorphous form of citrate |
| 2 | About 8 mg of compound I was weighed out and dissolved in 0.2 mL of acetone, and a citric acid solution (2 mol/L, 9.8 µL) was added. The compound was slurried overnight at room temperature, and 0.6 mL of isopropyl ether was added. The mixture was stirred for precipitation. | Amorphous form of citrate |
| 3 | About 8 mg of compound I was weighed out and dissolved in 0.2 mL of tetrahydrofuran, and a citric acid solution (2 mol/L, 9.8 µL) was added. The compound was slurried overnight at room temperature, and 0.6 mL of isopropyl ether was added. The mixture was stirred for precipitation. | Amorphous form of citrate |

### Example 54. Preparation of Amorphous Form of Malate

About 8 mg of compound I was weighed out and dissolved in 0.2 mL of ethanol, and a malic acid solution (2 mol/L, 9.8 µL) was added. The compound was slurried overnight at room temperature, and 0.6 mL of isopropyl ether was added. The mixture was stirred for precipitation and centrifuged, and the resulting solid was dried *in vacuo* to give a product.

The product was the amorphous form of the malate, as identified by X-ray powder diffraction.

### Example 55. Preparation of Amorphous Form of Malate

Compound I was weighed out, and a solvent and a 2 mol/L malic acid solution were added. Crystallization was performed to give a product. The crystal form was determined by X-ray powder diffraction, as shown in Table 35.

**Table 35. Preparation of the amorphous form of the malate**

| No. | Feed amount and solvent | Crystal form |
|---|---|---|
| 1 | About 8 mg of compound I was weighed out and dissolved in 0.2 mL of acetone, and a malic acid solution (2 mol/L, 9.8 µL) was added. The compound was slurried overnight at room temperature, and 0.6 mL of isopropyl ether was added. The mixture was stirred for precipitation. | Amorphous form of malate |
| 2 | About 8 mg of compound I was weighed out and dissolved in 0.2 mL of tetrahydrofuran, and a malic acid solution (2 mol/L, 9.8 µL) was added. The compound was slurried overnight at room temperature, and 0.6 mL of isopropyl ether was added. The mixture was stirred for precipitation. | Amorphous form of malate |
| 3 | About 8 mg of compound I was weighed out. 0.2 mL of acetonitrile was added, and a malic acid solution (2 mol/L, 9.8 µL) was added. The mixture was stirred for precipitation. | Amorphous form of malate |

### Example 56. Preparation of Amorphous Form of Hydrobromide

About 8 mg of compound I was weighed out. 0.2 mL of acetonitrile was added, and a hydrobromic acid solution (2 mol/L, 9.8 µL) was added. The compound was slurried overnight at room temperature, and 0.4 mL of isopropyl ether was added. The mixture was stirred for precipitation and centrifuged, and the resulting solid was dried *in vacuo* to give a product.

The product was the amorphous form of the hydrobromide, as identified by X-ray powder diffraction.

### Example 57. Preparation of Amorphous Form of Hydrobromide

Compound I was weighed out, and a solvent and a 2 mol/L hydrobromic acid solution were added. Crystallization was performed to give a product. The crystal form was determined by X-ray powder diffraction, as shown in Table 36.

**Table 36. Preparation of the amorphous form of the hydrobromide**

| No. | Feed amount and solvent | Crystal form |
|---|---|---|
| 1 | About 8 mg of compound I was weighed out. 0.2 mL of acetone was added, and hydrobromic acid (2 mol/L, 9.8 µL) was added. The compound was slurried overnight at room temperature, and 0.4 mL of isopropyl ether was added. The mixture was stirred for precipitation. | Amorphous form of hydrobromide |
| 2 | About 8 mg of compound I was weighed out. 0.2 mL of tetrahydrofuran was added, and hydrobromic acid (2 mol/L, 9.8 µL) was added. The compound was slurried overnight at room temperature, and 0.4 mL of isopropyl ether was added. The mixture was stirred for precipitation. | Amorphous form of hydrobromide |

### Example 58. Preparation of Amorphous Form of Mesylate

About 8 mg of compound I was weighed out. 0.2 mL of acetonitrile was added, and a mesylic acid solution (2 mol/L, 9.8 µL) was added. The compound was slurried overnight at room temperature, and 0.3 mL of isopropyl ether was added. The mixture was stirred for precipitation and centrifuged, and the resulting solid was dried *in vacuo* to give a product.

The product was the amorphous form of the mesylate, as identified by X-ray powder diffraction.

### Example 59. Preparation of Amorphous Form of Mesylate

Compound I was weighed out, and a solvent and a 2 mol/L mesylic acid solution were added. Crystallization was performed to give a product. The crystal form was determined by X-ray powder diffraction, as shown in Table 37.

**Table 37. Preparation of the amorphous form of the mesylate**

| No. | Feed amount and solvent | Crystal form |
|---|---|---|
| 1 | About 8 mg of compound I was weighed out. 0.2 mL of acetone was added, and mesylic acid (2 mol/L, 9.8 µL) was added. The compound was slurried overnight at room temperature, and 0.3 mL of isopropyl ether was added. The mixture was stirred for precipitation. | Amorphous form of mesylate |
| 2 | About 8 mg of compound I was weighed out. 0.2 mL of tetrahydrofuran was added, and mesylic acid (2 mol/L, 9.8 µL) was added. The compound was slurried overnight at room temperature, and 0.3 mL of isopropyl ether was added. The mixture was stirred for precipitation. | Amorphous form of mesylate |
| 3 | About 8 mg of compound I was weighed out. 0.2 mL of ethanol was added, and mesylic acid (2 mol/L, 9.8 µL) was added. The compound was slurried overnight at room temperature, and 0.3 mL of isopropyl ether was added. The mixture was stirred for precipitation. | Amorphous form of mesylate |

### Example 60. Preparation of Amorphous Form of Hydrochloride

About 8 mg of compound I was weighed out and dissolved in 0.2 mL of ethanol, and a hydrochloric acid solution (2 mol/L, 9.8 µL) was added. The mixture was stirred overnight, and 0.8 mL of isopropyl ether was added. The mixture was stirred for precipitation and centrifuged, and the resulting solid was dried *in vacuo* to give the title product.

The product was the amorphous form of the hydrochloride, as identified by X-ray powder diffraction.

### Experimental Example 1. Study on Influencing Factor Stability of Crystal Form I of p-Toluenesulfonate

The crystal form I of the p-toluenesulfonate was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 38. The influencing factor stability of the crystal form I of the p-toluenesulfonate**

| Conditions | Time (days) | Color and state | Purity% | Salt form |
|---|---|---|---|---|
| Initial | 0 | White solid | 100.0 | Crystal form I of p-toluenesulfonate |
| Light exposure (4500 Lux) | 5 | White solid | 99.9 | No change |
| | 10 | White solid | 99.9 | No change |
| | 30 | White solid | 99.7 | No change |
| 40 °C | 5 | White solid | 99.8 | No change |
| | 10 | White solid | 99.7 | No change |
| | 30 | White solid | 99.5 | No change |
| 60 °C | 5 | White solid | 99.8 | No change |
| | 10 | White solid | 99.8 | No change |
| | 30 | White solid | 99.7 | No change |
| 75% RH | 5 | White solid | 99.9 | No change |
| | 10 | White solid | 99.9 | No change |
| | 30 | White solid | 99.9 | No change |
| 92.5% RH | 5 | White solid | 100.0 | No change |
| | 10 | White solid | 100.0 | No change |
| | 30 | White solid | 99.9 | No change |

| | | | | |
|---|---|---|---|---|
| Conclusion: Standing under light exposure, high temperature (40 °C and 60 °C), and high humidity (75% and 92.5%) conditions for 30 days, the crystal form I of the p-toluenesulfonate exhibited good physical and chemical stability. | | | | |

### Experimental Example 2. Study on Influencing Factor Stability of Crystal Form I of Fumarate

The crystal form I of the fumarate was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 39. The influencing factor stability of the crystal form I of the fumarate**

| Conditions | Time (days) | Color and state | Purity% | Salt form |
|---|---|---|---|---|
| Initial | 0 | White solid | 100.0 | Crystal form I of fumarate |
| Light exposure (4500 Lux) | 5 | White solid | 100.0 | No change |
| | 10 | White solid | 99.9 | No change |
| | 30 | White solid | 99.5 | No change |
| 40 °C | 5 | White solid | 99.7 | No change |
| | 10 | White solid | 99.6 | No change |
| | 30 | White solid | 99.3 | No change |
| 60 °C | 5 | White solid | 99.8 | No change |
| | 10 | White solid | 99.7 | No change |
| | 30 | White solid | 99.5 | No change |
| 75% RH | 5 | White solid | 100.0 | No change |
| | 10 | White solid | 99.9 | No change |
| | 30 | White solid | 100.0 | No change |
| 92.5% RH | 5 | White solid | 99.9 | No change |
| | 10 | White solid | 99.9 | No change |
| | 30 | White solid | 99.9 | No change |

| | | | | |
|---|---|---|---|---|
| Conclusion: Standing under light exposure, high temperature (40 °C and 60 °C), and high humidity (75% and 92.5%) conditions for 30 days, the crystal form I of the fumarate exhibited good physical and chemical stability. | | | | |

Experimental Example 3. Study on Influencing Factor Stability of Crystal Form II of Hydrochloride The crystal form II of the hydrochloride was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 40. The influencing factor stability of the crystal form II of the hydrochloride**

| Conditions | Time (days) | Color and state | Purity% | Salt form |
|---|---|---|---|---|
| Initial | 0 | White solid | 100.0 | Crystal form II of hydrochloride |
| Light exposure (4500 Lux) | 5 | White solid | 99.8 | No change |
| | 10 | White solid | 99.2 | No change |
| | 30 | White solid | 96.5 | No change |
| 40 °C | 5 | White solid | 99.4 | No change |
| | 10 | White solid | 99.3 | No change |
| | 30 | White solid | 98.6 | No change |
| 60 °C | 5 | White solid | 99.4 | No change |
| | 10 | White solid | 98.8 | No change |
| | 30 | White solid | 97.4 | No change |
| 75% RH | 5 | White solid | 100.0 | No change |
| | 10 | White solid | 100.0 | No change |
| | 30 | White solid | 100.0 | No change |
| 92.5% RH | 5 | White solid | 100.0 | No change |
| | 10 | White solid | 100.0 | No change |
| | 30 | White solid | 100.0 | No change |

| | | | | |
|---|---|---|---|---|
| Conclusion: Standing under light exposure, high temperature (40 °C and 60 °C), and high humidity (75% and 92.5%) conditions for 30 days, the crystal form II of the hydrochloride exhibited good physical stability; the crystal form II of the hydrochloride exhibited good chemical stability under high humidity conditions. | | | | |

Experimental Example 4. Study on Influencing Factor Stability of Crystal Form III of Hydrochloride The crystal form III of the hydrochloride was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 41. The influencing factor stability of the crystal form III of the hydrochloride**

| Conditions | Time (days) | Color and state | Purity% | Salt form |
|---|---|---|---|---|
| Initial | 0 | White solid | 99.6 | Crystal form III of hydrochloride |
| Light exposure (4500 Lux) | 5 | White solid | 99.2 | No change |
| | 10 | White solid | 98.4 | No change |
| | 30 | White solid | 96.2 | No change |
| 40 °C | 5 | White solid | 99.6 | No change |
| | 10 | White solid | 99.4 | No change |
| | 30 | White solid | 99.0 | No change |
| 60 °C | 5 | White solid | 99.3 | No change |
| | 10 | White solid | 99.1 | No change |
| | 30 | White solid | 98.5 | No change |
| 75% RH | 5 | White solid | 99.6 | No change |
| | 10 | White solid | 99.6 | No change |
| | 30 | White solid | 99.6 | No change |
| 92.5% RH | 5 | White solid | 99.6 | No change |
| | 10 | White solid | 99.6 | No change |
| | 30 | White solid | 99.6 | No change |

| | | | | |
|---|---|---|---|---|
| Conclusion: Standing under light exposure, high temperature (40 °C and 60 °C), and high humidity (75% and 92.5%) conditions for 30 days, the crystal form III of the hydrochloride exhibited good physical stability; the crystal form III of the hydrochloride exhibited good chemical stability under high humidity conditions. | | | | |

Experimental Example 5. Study on Influencing Factor Stability of Crystal Form II of Phosphate The crystal form II of the phosphate was spread out and left to stand in open flasks, and the samples were subjected to 30-day stability tests under light exposure (4500 Lux), high temperature (40 °C and 60 °C), and high humidity (RH 75% and RH 92.5%) conditions.

**Table 42. The influencing factor stability of the crystal form II of the phosphate**

| Conditions | Time (days) | Color and state | Purity% | Salt form |
|---|---|---|---|---|
| Initial | 0 | White solid | 99.9 | Crystal form II of phosphate |
| Light exposure (4500 Lux) | 5 | White solid | 99.5 | No change |
| | 12 | White solid | 98.1 | No change |
| | 30 | White solid | 97.6 | No change |
| 40 °C | 5 | White solid | 99.4 | No change |
| | 12 | White solid | 99.1 | No change |
| | 30 | White solid | 98.7 | No change |
| 60 °C | 5 | White solid | 98.9 | No change |
| | 12 | White solid | 98.3 | No change |
| | 30 | White solid | 97.7 | No change |
| 75% RH | 5 | White solid | 99.8 | No change |
| | 12 | White solid | 99.7 | No change |
| | 30 | White solid | 99.5 | No change |
| 92.5% RH | 5 | White solid | 99.8 | No change |
| | 12 | White solid | 99.6 | Changed |
| | 30 | White solid | 99.4 | Changed |

| | | | | |
|---|---|---|---|---|
| Conclusion: The crystal form II of the phosphate exhibited good chemical stability under high humidity conditions and good physical stability under high temperature, light exposure, and high humidity (75% RH) conditions. | | | | |

### Experimental Example 6. Study on Long-Term Accelerated Stability of Crystal Form I of p-Toluenesulfonate

The crystal form I of the p-toluenesulfonate was sealed and left to stand under 25 °C/60% RH and 40 °C/75% RH conditions to test its stability.

**Table 43. The long-term/accelerated stability of the crystal form I of the p-toluenesulfonate**

| Test conditions | Time (months) | Purity (%) | Salt form |
|---|---|---|---|
| Initial | | 100.0 | Crystal form I of p-toluenesulfonate |
| 25°C, 60% RH | 1 | 100.0 | No change |
| | 2 | 100.0 | No change |
| | 3 | 100.0 | No change |
| | 6 | 100.0 | No change |
| 40 °C, 75% RH | 1 | 100.0 | No change |
| | 2 | 99.8 | No change |
| | 3 | 99.8 | No change |
| | 6 | 99.7 | No change |

| | | | |
|---|---|---|---|
| Conclusion: The long-term accelerated experiments show that standing under 25 °C/60% RH and 40 °C/75% RH conditions for 6 months, the crystal form I of the p-toluenesulfonate exhibited relatively good physical and chemical stability. | | | |

Experimental Example 7. Study on Long-Term Accelerated Stability of Crystal Form I of Fumarate The crystal form I of the fumarate was sealed and left to stand under 25 °C/60% RH and 40 °C/75% RH conditions to test its stability.

**Table 44. The long-term/accelerated stability of the crystal form I of the fumarate**

| Test conditions | Time (months) | Purity (%) | Salt form |
|---|---|---|---|
| Initial | | 100.0 | Crystal form I of fumarate |
| 25 °C, 60% RH | 1 | 99.9 | No change |
| | 2 | 100.0 | No change |
| | 3 | 100.0 | No change |
| | 6 | 99.9 | No change |
| 40 °C, 75% RH | 1 | 99.9 | No change |
| | 2 | 99.9 | No change |
| | 3 | 99.9 | No change |
| | 6 | 99.8 | No change |

| | | | |
|---|---|---|---|
| Conclusion: The long-term accelerated experiments show that standing under 25 °C/60% RH and 40 °C/75% RH conditions for 6 months, the crystal form I of the fumarate exhibited good physical and chemical stability. | | | |

### Experimental Example 8. Study on Long-Term Accelerated Stability of Crystal Form II of Hydrochloride

The crystal form II of the hydrochloride was sealed and left to stand under 25 °C/60% RH and 40 °C/75% RH conditions to test its stability.

**Table 45. The long-term/accelerated stability of the crystal form II of the hydrochloride**

| Test conditions | Time (months) | Purity (%) | Salt form |
|---|---|---|---|
| Initial | | 100.0 | Crystal form II of hydrochloride |
| 25 °C, 60% RH | 1 | 100.0 | No change |
| | 2 | 100.0 | No change |
| | 3 | 100.0 | No change |
| | 6 | 100.0 | No change |
| 40 °C, 75% RH | 1 | 100.0 | No change |
| | 2 | 100.0 | No change |
| | 3 | 99.9 | No change |
| | 6 | 99.9 | No change |

| | | | |
|---|---|---|---|
| Conclusion: The long-term accelerated experiments show that standing under 25 °C/60% RH and 40 °C/75% RH conditions for 6 months, the crystal form II of the hydrochloride exhibited good physical and chemical stability. | | | |

### Experimental Example 9. Study on Long-Term Accelerated Stability of Crystal Form III of Hydrochloride

The crystal form III of the hydrochloride was sealed and left to stand under 25 °C/60% RH and 40 °C/75% RH conditions to test its stability.

**Table 46. The long-term/accelerated stability of the crystal form III of the hydrochloride**

| Test conditions | Time (months) | Purity (%) | Salt form |
|---|---|---|---|
| Initial | | 99.6 | Crystal form III of hydrochloride |
| 25 °C, 60% RH | 1 | 99.6 | No change |
| | 2 | 99.6 | No change |
| | 3 | 99.6 | No change |
| | 6 | 99.6 | No change |
| 40 °C, 75% RH | 1 | 99.6 | No change |
| | 2 | 99.5 | No change |
| | 3 | 99.5 | No change |
| | 6 | 99.4 | No change |

| | | | |
|---|---|---|---|
| Conclusion: The long-term accelerated experiments show that standing under 25 °C/60% RH and 40 °C/75% RH conditions for 6 months, the crystal form III of the hydrochloride exhibited good physical and chemical stability. | | | |

### Experimental Example 10. Study on Long-Term Accelerated Stability of Crystal Form II of Phosphate

The crystal form II of the phosphate was sealed and left to stand under 25 °C/60% RH and 40 °C/75% RH conditions to test its stability.

**Table 47. The long-term/accelerated stability of the crystal form II of the phosphate**

| Test conditions | Time (months) | Purity (%) | Salt form |
|---|---|---|---|
| Initial | | 99.9 | Crystal form II of phosphate |
| 25 °C, 60% RH | 1 | 99.6 | No change |
| | 2 | 99.4 | No change |
| 40 °C, 75% RH | 1 | 98.7 | No change |
| | 2 | 97.7 | No change |

| | | | |
|---|---|---|---|
| Conclusion: The long-term accelerated experiments show that standing under 25 °C/60% RH and 40 °C/75% RH conditions for 2 months, the crystal form II of the phosphate exhibited relatively good physical stability and good long-term chemical stability. | | | |

## Claims

1. A pharmaceutically acceptable salt of 4-((1S,3 S,5R)-3-ethoxy-8-((5-methoxy-7-methyl-1H-indol-4-yl)methyl)-8-azabicyclo[3.2.1]octan-1-yl)benzoic acid, wherein the pharmaceutically acceptable salt is selected from the group consisting of a maleate, a phosphate, a p-toluenesulfonate, a sulfate, a hydrochloride, a fumarate, a tartrate, a succinate, a citrate, a malate, a mesylate, and a hydrobromide.

2. A preparation method for the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, comprising a step of reacting 4-((1S,3S,5R)-3-ethoxy-8-((5-methoxy-7-methyl-1H-indol-4-yl)methyl)-8-azabicyclo[3.2.1]octan-1-yl)benzoic acid with an acid, wherein the acid is selected from the group consisting of maleic acid, phosphoric acid, p-toluenesulfonic acid, sulfuric acid, hydrochloric acid, fumaric acid, tartaric acid, succinic acid, citric acid, malic acid, methanesulfonic acid, and hydrobromic acid.

3. The pharmaceutically acceptable salt according to claim 1, wherein a chemical ratio of the 4-((1S,3S,5R)-3-ethoxy-8-((5-methoxy-7-methyl-1H-indol-4-yl)methyl)-8-azabicyclo[3.2.1]octan-1-yl)benzoic acid to the acid is 3:1-1:2, preferably 2:1-1:1.

4. A crystal form I of the maleate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.7, 7.6, 8.6, 11.0, 12.1, and 16.2, preferably at 6.7, 7.6, 8.1, 8.6, 11.0, 12.1, 16.2, 19.7, and 23.5, and more preferably at 6.7, 7.6, 8.1, 8.6, 9.3, 11.0, 12.1, 13.5, 16.2, 17.9, 19.7, and 23.5; most preferably, the X-ray powder diffraction pattern expressed in terms of 2θ angles, which are diffraction angles, is shown in FIG. 1.

5. A crystal form I of the phosphate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 8.4, 10.3, 11.7, 14.8, 19.2, and 21.8, preferably at 8.4, 10.3, 11.7, 12.5, 14.8, 19.2, 19.8, 21.8, and 23.9, and more preferably at 7.0, 8.4, 9.3, 10.3, 11.7, 12.5, 14.8, 17.4, 19.2, 19.8, 21.8, and 23.9; most preferably, the X-ray powder diffraction pattern expressed in terms of 2θ angles, which are diffraction angles, is shown in FIG. 2.

6. A crystal form II of the phosphate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 8.4, 9.5, 10.2, 11.7, 14.7, and 19.1, preferably at 6.9, 8.4, 9.5, 10.2, 10.7, 11.7, 14.7, 18.5, and 19.1, and more preferably at 6.9, 8.4, 8.8, 9.5, 10.2, 10.7, 11.7, 14.7, 15.7, 18.5, 19.1, and 19.8; most preferably, the X-ray powder diffraction pattern expressed in terms of 2θ angles, which are diffraction angles, is shown in FIG. 3.

7. A crystal form III of the phosphate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 7.0, 8.0, 9.8, 11.5, 18.5, and 21.3, preferably at 7.0, 8.0, 9.8, 11.5, 16.1, 18.0, 18.5, 21.3, and 24.1, and more preferably at 7.0, 8.0, 9.8, 11.5, 16.1, 18.0, 18.5, 20.8, 21.3, 22.9, 24.1, and 25.3; most preferably, the X-ray powder diffraction pattern expressed in terms of 2θ angles, which are diffraction angles, is shown in FIG. 4.

8. A crystal form IV of the phosphate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.9, 8.4, 10.3, 11.7, and 14.8; preferably, the X-ray powder diffraction pattern expressed in terms of 2θ angles, which are diffraction angles, is shown in FIG. 5.

9. A crystal form V of the phosphate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 9.1, 10.2, 11.5, 15.7, and 19.8, preferably at 8.6, 9.1, 10.2, 11.5, 15.7, 18.0, 19.8, and 23.5; more preferably, the X-ray powder diffraction pattern expressed in terms of 2θ angles, which are diffraction angles, is shown in FIG. 6.

10. A crystal form I of the p-toluenesulfonate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.0, 9.4, 10.1, 16.3, and 18.3, preferably at 5.0, 9.4, 10.1, 16.3, 18.3, 18.9, 21.2, and 22.9, and more preferably at 5.0, 9.4, 10.1, 16.0, 16.3, 17.1, 18.3, 18.9, 21.2, 22.9, and 24.0; most preferably, the X-ray powder diffraction pattern expressed in terms of 2θ angles, which are diffraction angles, is shown in FIG. 7.

11. A crystal form II of the p-toluenesulfonate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 4.7, 8.8, 9.3, 10.8, 13.9, and 18.7, preferably at 4.7, 8.8, 9.3, 9.7, 10.8, 13.9, 17.7, and 18.7; more preferably, the X-ray powder diffraction pattern expressed in terms of 2θ angles, which are diffraction angles, is shown in FIG. 8.

12. A crystal form III of the p-toluenesulfonate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.8, 7.4, 8.1, 10.1, and 12.7; preferably, the X-ray powder diffraction pattern expressed in terms of 2θ angles, which are diffraction angles, is shown in FIG. 9.

13. A crystal form I of the sulfate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 7.2, 9.2, 17.1, 20.0, 21.4, and 24.7, preferably at 6.7, 7.2, 9.2, 17.1, 18.7, 20.0, 21.4, 22.9, and 24.7; more preferably, the X-ray powder diffraction pattern expressed in terms of 2θ angles, which are diffraction angles, is shown in FIG. 10.

14. A crystal form II of the sulfate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 9.5, 10.2, 16.6, 21.2, and 25.7, preferably at 6.3, 8.5, 9.5, 10.2, 16.6, 19.8, 21.2, 23.7, and 25.7; more preferably, the X-ray powder diffraction pattern expressed in terms of 2θ angles, which are diffraction angles, is shown in FIG. 11.

15. A crystal form III of the sulfate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.9, 7.6, 9.1, 18.2, and 23.7, preferably at 6.9, 7.6, 9.1, 17.0, 18.2, 20.7, 23.7, and 24.0; more preferably, the X-ray powder diffraction pattern expressed in terms of 2θ angles, which are diffraction angles, is shown in FIG. 12.

16. A crystal form IV of the sulfate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.9, 12.5, 16.5, 19.4, 21.2, and 24.0, preferably at 6.9, 9.7, 12.5, 16.5, 19.4, 21.2, 24.0, and 25.8; more preferably, the X-ray powder diffraction pattern expressed in terms of 2θ angles, which are diffraction angles, is shown in FIG. 13.

17. A crystal form V of the sulfate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 7.6, 11.4, 13.5, 17.2, 18.8, and 19.5, preferably at 7.6, 10.0, 11.4, 13.5, 14.0, 17.2, 19.5, 22.5, and 24.6; more preferably, the X-ray powder diffraction pattern expressed in terms of 2θ angles, which are diffraction angles, is shown in FIG. 14.

18. A crystal form VI of the sulfate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.7, 8.8, 14.6, 15.9, and 23.7, preferably at 6.7, 8.8, 10.6, 14.6, 15.9, 19.5, 21.4, and 23.7; more preferably, the X-ray powder diffraction pattern expressed in terms of 2θ angles, which are diffraction angles, is shown in FIG. 15.

19. A crystal form I of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.8, 8.8, 11.6, 20.7, and 23.4, preferably at 5.8, 8.8, 9.8, 10.5, 11.6, 14.6, 18.4, 20.7, and 23.4; more preferably, the X-ray powder diffraction pattern expressed in terms of 2θ angles, which are diffraction angles, is shown in FIG. 16.

20. A crystal form II of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.9, 8.8, 10.6, 17.2, 19.3, and 23.9, preferably at 5.9, 8.8, 10.6, 13.2, 17.2, 19.3, 21.3, 23.9, 24.4, and 26.1, and more preferably at 5.9, 8.8, 10.6, 11.9, 13.2, 14.7, 17.2, 19.3, 19.9, 21.3, 23.9, 24.4, 26.1, and 27.4; most preferably, the X-ray powder diffraction pattern expressed in terms of 2θ angles, which are diffraction angles, is shown in FIG. 17.

21. A crystal form III of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.1, 8.8, 10.4, 18.4, 19.9, and 24.6, preferably at 6.1, 8.8, 10.4, 12.2, 18.4, 19.9, 22.6, 24.6, and 28.0, and more preferably at 6.1, 8.8, 10.4, 12.2, 14.6, 16.6, 17.8, 18.4, 19.9, 22.6, 24.6, 27.2, and 28.0; most preferably, the X-ray powder diffraction pattern expressed in terms of 2θ angles, which are diffraction angles, is shown in FIG. 18.

22. A crystal form IV of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.4, 9.0, 10.8, 20.4, and 21.8, preferably at 5.4, 9.0, 10.8, 19.3, 20.4, 21.8, and 27.3; more preferably, the X-ray powder diffraction pattern expressed in terms of 2θ angles, which are diffraction angles, is shown in FIG. 19.

23. A crystal form V of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.2, 6.7, 7.7, 10.2, and 17.4, preferably at 5.2, 6.7, 7.7, 10.2, 10.8, 17.4, 20.5, and 24.2; more preferably, the X-ray powder diffraction pattern expressed in terms of 2θ angles, which are diffraction angles, is shown in FIG. 20.

24. A crystal form VI of the hydrochloride of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 5.8, 10.3, 11.7, 17.7, 20.7, and 23.7; preferably, the X-ray powder diffraction pattern expressed in terms of 2θ angles, which are diffraction angles, is shown in FIG. 21.

25. A crystal form I of the fumarate of 4-((1S,3 S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 9.6, 14.0, 16.7, 19.6, 25.8, and 26.1, preferably at 6.1, 9.6, 10.0, 14.0, 16.7, 17.2, 19.1, 19.6, 25.8, and 26.1, and more preferably at 6.1, 9.6, 10.0, 10.8, 14.0, 16.7, 17.2, 18.6, 19.1, 19.6, 20.2, 25.8, and 26.1; most preferably, the X-ray powder diffraction pattern expressed in terms of 2θ angles, which are diffraction angles, is shown in FIG. 22.

26. A crystal form II of the fumarate of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 6.2, 6.6, 8.0, 13.2, 14.0, 20.3, and 24.2, preferably at 6.2, 6.6, 8.0, 9.0, 13.2, 14.0, 16.4, 17.1, 19.8, 20.3, 24.2, and 25.3, and more preferably at 6.2, 6.6, 8.0, 9.0, 12.0, 13.2, 14.0, 16.4, 17.1, 19.3, 19.8, 20.3, 21.9, 22.3, 24.2, 25.3, 25.7, and 28.1; most preferably, the X-ray powder diffraction pattern expressed in terms of 2θ angles, which are diffraction angles, is shown in FIG. 23.

27. A crystal form I of the hydrobromide of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 7.6, 10.6, 16.4, 18.4, 22.6, and 24.0, preferably at 7.6, 10.6, 15.3, 16.4, 18.4, 19.6, 22.6, 24.0, 26.5, and 27.0, and more preferably at 7.6, 10.6, 15.3, 16.4, 18.4, 19.6, 21.4, 22.6, 24.0, 25.5, 26.5, 27.0, and 28.9; most preferably, the X-ray powder diffraction pattern expressed in terms of 2θ angles, which are diffraction angles, is shown in FIG. 24.

28. A crystal form II of the hydrobromide of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1, wherein an X-ray powder diffraction pattern, expressed in terms of 2θ angles, which are diffraction angles, of the crystal form has characteristic peaks at 7.2, 10.5, 16.5, 22.5, 23.4, and 26.6, preferably at 7.2, 10.5, 13.1, 16.5, 18.8, 20.3, 22.5, 23.4, and 26.6, and more preferably at 7.2, 10.5, 13.1, 16.5, 17.2, 18.8, 20.3, 21.5, 21.9, 22.5, 23.4, and 26.6; most preferably, the X-ray powder diffraction pattern expressed in terms of 2θ angles, which are diffraction angles, is shown in FIG. 25.

29. The crystal form of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to any one of claims 4-26, wherein the 2θ angles have a margin of error of ±0.2.

30. A pharmaceutical composition, comprising the following components:
i) the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1 or the crystal form of the pharmaceutically acceptable salt of the compound represented by formula (I) according to any one of claims 4-22; and
ii) one or more pharmaceutically acceptable excipients.

31. A method for preparing a pharmaceutical composition, comprising a step of mixing the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1 or the crystal form of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to any one of claims 4-29 with a pharmaceutically acceptable excipient.

32. Use of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1 or the crystal form of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to any one of claims 4-29, or the composition according to claim 30 in the preparation of a medicament for inhibiting activation of the complement alternative pathway, preferably in the preparation of a medicament for inhibiting complement factor B.

33. Use of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to claim 1 or the crystal form of the pharmaceutically acceptable salt of 4-((1S,3S,5R)-3-ethoxy-8-((5-methoxy-7-methyl-1H-indol-4-yl)methyl)-8-azabicyclo[3.2.1]octan-1-yl)benzoic acid according to any one of claims 4-29, or the composition according to claim 30 in the preparation of a medicament for treating a disease or disorder, wherein the disease or disorder is selected from the group consisting of glomerulopathy, hemolytic uremic syndrome, atypical haemolytic uraemic syndrome, paroxysmal nocturnal hemoglobinuria, age-related macular degeneration, geographic atrophy, diabetic retinopathy, uveitis, retinitis pigmentosa, macular edema, Behcet's uveitis, multifocal choroiditis, Vogt-Koyanagi-Harada syndrome, birdshot retino-chorioditis, sympathetic ophthalmia, ocular dicatricial pemphigoid, ocular pemphigus, nonartertic ischemic optic neuropathy, post-operative inflammation, retinal vein occlusion, neurological disorders, multiple sclerosis, stroke, Guillain-Barré syndrome, traumatic brain injury, Parkinson's disease, disorders of inappropriate or undesirable complement activation, hemodialysis complications, hyperacute allograft rejection, xenograft rejection, interleukin-2 induced toxicity during IL-2 therapy, Crohn's disease, adult respiratory distress syndrome, myocarditis, post-ischemic reperfusion conditions, myocardial infarction, balloon angioplasty, post-pump syndrome in cardiopulmonary bypass or renal bypass, atherosclerosis, hemodialysis, renal ischemia, mesenteric artery reperfusion after aortic reconstruction, infectious disease or sepsis, systemic lupus erythematosus, systemic lupus erythematosus nephritis, proliferative nephritis, liver fibrosis, hemolytic anemia, myasthenia gravis, tissue regeneration, neural regeneration, dyspnea, hemoptysis, acute respiratory distress syndrome, asthma, chronic obstructive pulmonary disease, emphysema, pulmonary embolisms and infarcts, pneumonia, fibrogenic dust diseases, pulmonary fibrosis, asthma, allergy, bronchoconstriction, parasitic diseases, Goodpasture's syndrome, pulmonary vasculitis, pauci-immune vasculitis, immune complex-associated inflammation, antiphospholipid syndrome, and obesity; the disease or disorder is preferably C3 glomerulopathy, IgA nephropathy, membranous glomerulonephritis, atypical haemolytic uraemic syndrome, and paroxysmal nocturnal hemoglobinuria.
